(19) Europäisches Patentamt
European Patent Office
Office européen des brevets

(11) **EP 3 372 988 B1**

(12) **EUROPÄISCHE PATENTSCHRIFT**

(45) Veröffentlichungstag und Bekanntmachung des Hinweises auf die Patenterteilung:
**12.10.2022 Patentblatt 2022/41**

(21) Anmeldenummer: **17160250.1**

(22) Anmeldetag: **10.03.2017**

(51) Internationale Patentklassifikation (IPC):
*G01N 21/3504* $^{(2014.01)}$    *A61B 5/083* $^{(2006.01)}$
*G01N 33/497* $^{(2006.01)}$    *G01N 21/31* $^{(2006.01)}$
*G01N 33/00* $^{(2006.01)}$    *G01J 3/42* $^{(2006.01)}$
*G01J 3/02* $^{(2006.01)}$    *A61B 5/08* $^{(2006.01)}$
*G01J 3/10* $^{(2006.01)}$    *G01J 3/427* $^{(2006.01)}$
*G01J 3/12* $^{(2006.01)}$    *G01N 21/37* $^{(2006.01)}$

(52) Gemeinsame Patentklassifikation (CPC):
**G01N 21/3504; A61B 5/0836; G01J 3/0205; G01J 3/0216; G01J 3/10; G01J 3/42; G01J 3/427; G01N 21/314; G01N 21/37; G01N 33/497;**
G01J 2003/1213; G01N 2021/3536

(54) **VERFAHREN UND VORRICHTUNG ZUM MESSEN EINER STOFFKONZENTRATION IN EINEM GASFÖRMIGEN MEDIUM MITTELS ABSORPTIONSSPEKTROSKOPIE**

METHOD AND DEVICE FOR MEASURING THE CONCENTRATION OF A SUBSTANCE IN A GASEOUS MEDIUM BY MEANS OF ABSORPTION SPECTROSCOPY

PROCÉDÉ ET DISPOSITIF DE MESURE D'UNE CONCENTRATION DE MATIÈRE DANS UN MILIEU GAZEUX AU MOYEN DE LA SPECTROSCOPIE D'ABSORPTION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**12.09.2018 Patentblatt 2018/37**

(73) Patentinhaber: **Sensatronic GmbH**
**23970 Wismar (DE)**

(72) Erfinder:
• **DEGNER, Martin**
  **18209 Reddelich (DE)**
• **EWALD, Hartmut**
  **18055 Rostock (DE)**

(74) Vertreter: **Seemann & Partner Patentanwälte mbB**
**Raboisen 6**
**20095 Hamburg (DE)**

(56) Entgegenhaltungen:
**KR-A- 20160 063 704    US-A- 5 445 160**
**US-A1- 2011 147 592**

• **WANG X ET AL: "High performance CO2 measurement based on pressure modulation", PROCEDIA ENGINEERING, ELSEVIER, AMSTERDAM, NL, Bd. 5, 2010, Seiten 1208-1211, XP027483885, ISSN: 1877-7058, DOI: 10.1016/J.PROENG.2010.09.329 [gefunden am 2010-10-25]**

EP 3 372 988 B1

**Beschreibung**

Die Erfindung betrifft eine Anordnung und ein Verfahren zum Messen einer Gaskonzentration mittels Absorptionsspektroskopie, insbesondere zur kapnometrischen Messung des Anteils von $CO_2$ in Atemluft.

Das Grundprinzip solcher Messungen ist bekannt. Licht, oder, im Falle der Messung von $CO_2$, infrarotes (IR) Licht von einer thermischen Lichtquelle, wird durch eine Messzelle mit einem zu analysierenden Gasgemisch geleitet, und die Gaskonzentration eines in dem Gasgemisch enthaltenen zu messenden Gases über eine Messung einer Abschwächung des in die Messzelle eingeleiteten Lichts aufgrund von Absorption in dem zu messenden Gas bestimmt.

Die Abschwächung des ausgesandten Lichts hängt exponentiell von der Konzentration bzw. Dichte des absorbierenden Gases, dem wellenlängenabhängigen Absorptionskoeffizienten des Gases und der Länge der Messstrecke ab, in der das Licht das zu messende Gas durchquert. Die Messung der Abschwächung des Lichts durch Absorption setzt die Kenntnis der Menge an in die Messzelle eingestrahltem und aus der Messzelle ausgekoppeltem Licht voraus. Aus diesem Grund sind grundsätzlich zwei Messungen notwendig, nämlich einerseits die Messung der Lichtmenge des durch Absorption im Messgas abgeschwächten Lichts nach Durchquerung der Messzelle und andererseits als Referenzmessung die Messung einer Größe, die repräsentativ ist für die eingestrahlte Lichtmenge.

Diese Referenzmessung kann eine direkte Messung der Lichtintensität oder Lichtmenge an der Lichtquelle sein, wobei ein Lichtanteil der Quelle erfasst wird, welcher nicht für die Gasmessung genutzt wird, aber dennoch proportional zur Lichtmenge der Gasmessung sein soll. Der Vorteil hiervon ist eine direkte Messung der ausgestrahlten Lichtintensität, problematisch ist, dass das für die Referenzmessung verwendete Licht nur proportional und nicht identisch zu dem für die Gasmessung genutzten Licht ist. Die proportionale Abhängigkeit kann sich im Laufe der Zeit ändern, da das Licht der Referenzmessung nicht denselben optischen Pfad durchlaufen hat wie das Licht der Absorptionsmessung und somit zusätzliche Absorptionen, beispielsweise durch Verunreinigungen, nicht aufweist. Auch längerfristige Entwicklungen, wie beispielsweise eine zunehmende Verunreinigung der Messfenster der Messzelle, können zu einer systematischen Drift der Absorptionsmessergebnisse führen.

Eine alternative Anordnung verwendet Referenzlicht, welches ebenfalls die Messzelle durchquert, aber in einem Wellenlängenbereich liegt, in dem das zu messende Gas wenig oder nicht absorbiert. Das Referenzlicht erfährt somit nur die Absorption, die durch Verunreinigungen oder andere systematische Quellen hervorgerufen wird, und welcher auch das Licht im Wellenlängenbereich der Absorption durch das zu messende Gas unterliegt. Eine zunehmende Verschmutzung in der Messzelle beispielsweise wirkt sich dann, bis auf wellenlängenabhängige, also dispersive Effekte, in gleicher Weise auf die Absorptionsmessung wie auf die Referenzmessung aus. Bei dem Vergleich der beiden Messungen heben diese Anteile sich weitgehend auf, sodass entsprechende systematische Effekte, die das Messergebnis negativ beeinflussen, weitgehend minimiert werden.

Im Bereich der Kapnometrie wird infrarotes Licht verwendet, da das Gas $CO_2$ eine starke Absorptionsbande bei ca. $4,26\,\mu$m, also im mittleren Infrarotbereich, aufweist. Die vorliegende Erfindung ist daher im Bereich der Kapnometrie besonders anwendbar, jedoch auch auf andere Anwendungen anwendbar, bei denen Absorptionsmessungen, insbesondere im Infrarotbereich, durchgeführt werden. Die Kapnometrie ist ein in der Medizin angewandtes Überwachungsverfahren und liefert Aussagen zum Patientenzustand im Bereich des Rettungswesens, in Kliniken, der häuslichen Versorgung und der Sportmedizin.

Kapnometer sind meist kleine mobile Geräte, teilweise mit einer in der Hand tragbaren Einheit zur Parametrisierung und Visualisierung, wobei in vielen Fällen bei Seitenstromanwendungen die Sensoreinheit auch im Handgerät enthalten ist. Solche Kapnometer können auch als fertige Baugruppen in größeren Geräten, beispielsweise Beatmungsgeräten, metabolischen Monitoren unter anderem integriert werden.

Kapnometer erfassen den $CO_2$-Verlauf der Atmung eines Patienten. Diese Messung erfolgt entweder im sogenannten Hauptstromverfahren oder im sogenannten Seitenstromverfahren. Beim Hauptstromverfahren wird meist in der Nähe der Atemmaske des Patienten ein Sensor platziert, der durch eine Küvette im Atemschlauch die $CO_2$-Konzentration misst. Beim Seitenstromverfahren wird eine Küvette mit einem dünnen Absaugschlauch im Atemgaskanal angebracht, ein Teil der Atemluft durch den Absaugschlauch zu einem Sensormodul gefördert und dort mit gewisser Totzeit gemessen.

Aufgrund der Anwendungen, speziell im Bereich des Rettungswesens und der häuslichen Versorgung sowie der Sportmedizin, sollten Sensormodule klein, leicht und robust sein, um Erschütterungen standzuhalten, und eine geringe Leistungsaufnahme aufweisen, weil häufig ein mobiler Einsatz ohne externe Stromversorgung notwendig ist. Gegenwärtig verfügbare Hauptstromsensoren haben nur eine Akkulaufzeit von vier bis sechs Stunden. Für kapnometrische Messungen wird nach der Norm ISO 80601-2-55 eine Genauigkeit von $\pm0{,}43\%$ $CO_2$ + 8% des Messwerts gefordert. Am Markt befindliche Geräte übertreffen diese Anforderungen in der Regel deutlich. Ferner sollte zur Auflösung des Atemverlaufs auch bei hoher Atemfrequenz von bis zu 150 Atemzügen pro Minute, wie sie u.a. bei Frühgeborenen vorkommen können, ein Messintervall mit voneinander unabhängigen Messwerten von 10 ms bis 40 ms, vorzugsweise bis 25 ms, verwirklicht werden. Mit dieser zeitlichen Auflösung wird es möglich, den endexpiratorischen Wert, der eine medizinische Kenngröße ist, sicher zu ermitteln. Gerade für zeitkritische Anwendungen wie die Notfallmedizin ist auch eine schnelle Messbereitschaft nach einem Kaltstart, also eine geringe sogenannte "start-up-time", gewünscht. Hier benötigen herkömmliche Systeme teilweise mehrere Minuten, um die erforderliche Betriebstemperatur zu erreichen. Außerdem ist eine kostengünstige und einfache Herstellung gewünscht.

Bekannt sind beispielsweise Filterradanordnungen, wie sie von Massimo Inc. vertrieben werden und beispielsweise in WO 03/060490 A offenbart sind. Diese Anordnungen umfassen ein sich mechanisch drehendes Rad im optischen Strahlengang mit wenigstens zwei Filtern für Gasabsorption und Referenzwellenlänge, und zusätzlich mit einem Bereich, in dem das Licht vollständig abgeschottet wird. Die Abschottung ermöglicht eine Dunkelmessung, die zur Offsetkompensation der Empfangseinheit genutzt wird. Hier wird das Messprinzip verwirklicht, dass die Messwellenlänge und die Referenzwellenlänge den gleichen optischen Pfad durchqueren. Das Licht muss auch nicht auf unterschiedliche Pfade aufgeteilt werden. Es handelt sich um ein Zeitmultiplex-Messverfahren, und die Verwendung mechanisch bewegter Bauteile im optischen Strahlengang ist gegebenenfalls erschütterungsanfällig.

Eine Anordnung mit spektralem Strahlteiler ist beispielsweise aus US 5464982 A und US 5092342 A bekannt, wobei moduliertes Licht empfangsseitig auf zwei oder mehr Detektoren für die Gasabsorption und für die Referenzwellenlänge aufgeteilt wird. Auch hierbei handelt es sich um eine typische Spektroskopieanordnung, bei der eine Lichtquelle verwendet wird und eine hohe Lichtausbeute erzielt wird, da das Licht wellenlängenselektiv und nicht geometrisch aufgeteilt wird. Es treten jedoch Verluste bedingt durch die Dämpfung im Transmissionsbereich der Filter auf. Es erfolgt eine simultane Messung bei der Absorptionswellenlänge und der Referenzwellenlänge, sodass keine Einschränkungen bezüglich des Zeitverlaufs der Zielgröße vorhanden sind. Die Integration von mehr als zwei Wellenlängen zur Messung mehrerer Messgase ist verhältnismäßig aufwendig. Es kann ein Strahlteilerfilter erforderlich sein, der teuer sein kann. Im Interesse einer Offsetkompensation kann die Quelle in diesem Fall auch zusätzlich moduliert werden.

Ein weiterer grundsätzlicher Anordnungstyp ist die geometrische Parallelmessung. Hierbei wird das Licht der Lichtquelle ohne Strahlteilung auf mehrere ungefilterte Empfänger geführt. Dadurch werden für jeden Messpfad, also Gaspfad und Referenzpfad, unterschiedliche optische Lichtpfade genutzt. Dies bedeutet auch, dass bei der Absorptionsmessung und der Referenzmessung verschiedene Bereiche der Quelle und unterschiedliche Transmissionspfade durch die Messgeräte zum Einsatz kommen. Hieraus resultieren Doppeldeutigkeiten, welchen Effekt die geometrische Teilung und welchen Effekt die eigentliche Absorption im Messgas auf die Messung hat. Diese Doppeldeutigkeiten können systematische Messfehler erzeugen. Diese Anordnungen sind etwa aus US 4618771 A und US 6277081 B1 bekannt, werden in der Praxis jedoch seltener eingesetzt, beispielsweise durch Goldwei (China). Vorteile hierbei sind die recht einfache Anordnung, die simultane Messung von Absorption und Referenz sowie der Verzicht auf mechanisch bewegte Bauteile. Die geometrische Parallelmessung leidet allerdings unter einer schlechteren Störunterdrückung bezüglich räumlicher Schwankungen der Quelle, unterschiedlicher Lichtübertragung zu den beiden Detektoren, beispielsweise durch die Bildung von Kondensat in der Küvette, sowie durch eine reduzierte Lichtausbeute aufgrund der ungerichteten Beleuchtung der Empfänger. Auch in diesem Fall kann die Quelle im Interesse einer Offsetkompensation zusätzlich moduliert werden.

Ein weiteres Messprinzip beinhaltet einen Wellenlängenshift der Lichtquelle. Das Maximum des Emissionsspektrums einer meist schmalbandigen Lichtquelle wird, meist thermisch induziert, zwischen Bereichen starker und schwacher Absorption der Zielgröße, beispielsweise $CO_2$, zeitlich alternierend moduliert. Somit stehen in der zeitlichen Abfolge der Messwerte alternierend Werte mit hohem Einfluss der Zielgröße (Gasmessung) und mit niedrigem Einfluss (Referenzmessung) zur Verfügung. Typischerweise werden für diese Art der Messung Laser als Lichtquellen eingesetzt, beispielsweise Diodenlaser. Im Bereich der $CO_2$-Klimamesstechnik hat sich auch ein Messverfahren etabliert, bei dem eine LED spektral moduliert wird, wie beispielsweise in WO 2007/091043 A1 beschrieben. Dieses Verfahren benutzt nur einen optischen Pfad, eine Lichtquelle und einen Detektor und erfordert keine optischen Filter, da die schmalbandige Lichtquelle spektral moduliert wird. Diese schmalbandigen Lichtquellen haben eine im Verhältnis zur benötigten Emission niedrige Leistungsaufnahme, jedoch kann die thermische Modulation dieser Lichtquellen zusätzlich sehr energieaufwändig sein. Es handelt sich um eine Zeitmultiplex-Messung, d. h., die Modulation des Emissionsspektrums muss schneller erfolgen als die geringste Zeitkonstante der Messgröße. In der Kapnometrie kann diese Einschränkung problematisch sein, im Bereich der Klimamesstechnik stellt dies kein Problem dar Die Ansteuerung, die Umsetzung des Messverfahrens und die Fertigung der Sensoren, insbesondere bei LEDs und Fotodioden, ist recht komplex. Ferner ist es schwierig, den für die Modulation erforderlichen großen Temperaturbereich auszufüllen, und die Messung ist anfällig bezüglich Temperaturgradienten. Durch die Modulation ist die Lichtausbeute reduziert, was sich negativ auf das Signal-Rausch-Verhältnis ("Signal-to-Noise-Ratio", SNR) der Messung auswirkt.

Bei der Kapnometrie hingegen werden überwiegend thermische Strahler als Lichtquelle für die optische $CO_2$-Messung im mittleren Infrarot (MIR) eingesetzt, da die Hauptabsorption von $CO_2$ bei ca. 4,26 $\mu$m liegt. Diese thermischen Strahler sind kostengünstig und robust. Lichtquellen in Kapnometrie-Anordnungen sollen eine hohe Emissionsleistung erreichen, um bei den Empfängern genügend Lichtstärke für ein gutes SNR zu ermöglichen. Detektoren im mittleren Infrarotbereich weisen häufig ein hohes thermisches Eigenrauschen auf, daher wird für ein gutes SNR viel Nutzsignal benötigt. Ebenfalls wird viel Nutzsignal benötigt, um die geforderte Konzentrationsauflösung bei der vorgegebenen kurzen Messzeit von 10 ms bis 25 ms zu erreichen.

Die Physik des thermischen Strahlers besagt, dass die emittierte Intensität nur von der Temperatur des Strahlers sowie von weiteren Konstanten wie der Stefan-Boltzmann-Konstante und dem Emissionsgrad abhängig ist. Eine Erhöhung der insgesamt abgegebenen optischen Leistung kann bei Nutzung einer maximal möglichen Temperatur der Lichtquelle und bei möglichst hohem Emissionsgrad nur durch Erhöhen der Emissionsfläche erreicht werden. Diese Parameter sind technisch limitiert. In der Praxis wird in der Kapnometrie typischerweise ein flächiger Filamentstrahler oder Flächenstrahler mit einer aktiven strahlenden Fläche von mehr als 1 mm$^2$ verwendet bei einer Emissionstemperatur, die häufig bei ca. 400 °C bis 600 °C liegt. So liegt bei einer Emissionstemperatur von ca. 410 °C das Maximum der Emission bei 4,26 $\mu$m, also der $CO_2$-Absorptionslinie. Zusätzlich wird durch diese vergleichsweise niedrige Temperatur die Korrosion des thermischen Strahlers begrenzt, was für die Langlebigkeit der Lichtquelle vorteilhaft ist, die häufig nicht durch einen evakuierten Glaskolben geschützt ist. Auf einen Glaskolben wird verzichtet, um zusätzliche Absorptionsverluste durch das Glas zu vermeiden.

Solche großflächigen Emitter ohne evakuierten Glaskolben erfordern allerdings viel elektrische Leistung, um die erforderliche Strahlertemperatur zu erreichen, bei Kapnometern typischerweise mehr als 300 mW. Außerdem führt ein großflächiger Emitter trotz Leistungsüberhöhung nach dem Start zu einer hohen Start-up Zeit, beispielsweise zwischen 30 und 150 Sekunden.

Die große thermische Masse macht den Emitter auch träge, so dass nur eine geringe Modulationsrate möglich ist, deren Zeitkonstante dann größer ist als die der Zielgröße. Dadurch kann bei AC-Betrieb zur Offsetkompensation insbesondere bei schneller Atmung nicht gemessen werden. Der endexpiratorische Wert wird nur noch ungenau bestimmt. Bei einem thermisch induzierten Wellenlängenshift ist die Funktionsweise dieses Messprinzips für die Umgebungsbedingungen in der Kapnometrie in Bezug auf den Temperaturbereich und Temperaturgradienten auch nur schwierig und nicht kostengünstig umzusetzen.

Eine weitere Eigenart thermischer Strahler (schwarzer Körper) ist, dass sie eine Lambert'sche Strahlungscharakteristik aufweisen, was in Kombination mit einer großen Emissionsfläche bedeutet, dass das Licht auch mit bündelnden Optiken nicht effizient und ohne Verluste auf einen oder mehrere Detektoren geleitet werden kann. Ein Großteil der erzeugten Strahlung wird somit nicht zur Messung genutzt, was energetisch ungünstig ist.

Die Kompensation der thermischen Drift der Empfänger und die Unterdrückung des Einflusses anderer thermischer Strahlungsquellen auf das Messsignal, beispielsweise die Gehäusetemperatur und die Sensorerwärmung, können im Rahmen eines sogenannten AC-Betriebs durch eine Amplitudenmodulation des Lichts bei der Transmissionsmessung erfolgen, oder eine Amplitudenmodulation wird genutzt, die durch ein Filterrad oder ein Chopper-Rad erzeugt wird. Die niedrige Modulationsfrequenz großflächiger thermischer Strahler ist bei elektronischer Modulation problematisch, da bei höheren Atemfrequenzen nur noch ungenau mitgemessen werden kann. Die mechanische Modulation ist hingegen mechanisch störanfällig. In anderen Ausführungsformen wird das Licht nicht moduliert. Hier resultieren deutliche Messfehler durch Drift und parasitäre thermische Strahlung. Solche Sensoren sind für die Kapnometrie nicht geeignet.

In entsprechenden Anwendungen werden meist Thermopile-Detektoren eingesetzt. Diese sind großflächig, um möglichst viel Licht im Aufbau zu erfassen. Sie sind jedoch auch recht verrauscht, sodass die erforderliche Signalfilterung zu niedrigen Grenzfrequenzen der Detektoren führt, wodurch ebenfalls die Frequenz im AC-Betrieb begrenzt wird. Dies betrifft auch die mögliche Rotationsgeschwindigkeit in einem Filterradaufbau. Alternativ werden üblicherweise pyroelektrische Detektoren verwendet, welche jedoch deutlich kostenintensiver sind.

Aus KR 2016 0063704 A ist eine weitere Gasmessungsvorrichtung bekannt, welche eine Infrarotlampe und einen für das Infrarot optimierten paraboloiden Reflektor umfasst, um das Infrarotlicht über eine große Distanz auf einen pyroelektrischen Sensor zu lenken, der ein feines Signal verstärkt.

Weiter ist aus US 5,445,160 A ein $CO_2$-Monitor bekannt. Der Monitor umfasst eine austauschbare Teilvorrichtung, die einen Luftweg-Sensor umfasst zur Verbindung zwischen einem Ventilatorauslass und einer endotrachialen Röhre. In einem Eingang davon ist eine Wegwerf-Infrarotlichtquelle eingelassen. Ein wiederverwendbarer Teil des Monitors ist ein Detektormodul, das einen Detektor und einen Verstärker umfasst. Das Detektormodul wird an den Luftweg-Sensor angeschlossen, so dass sich der Detektor in einem weiteren Ausgang des Sensors befindet.

Aus Wang, X. et al., "High Performance CO2 Measurement Based on Pressure Modulation", Procedia Engineering, Bd. 5 (2010), Seiten 1208-1211, ist bekannt, dass auf der Grundlage des Beer-Lambert'schen Gesetzes Druckmodulationen zur Erhöhung der Auflösung und zum Eliminieren von Langzeit-Drift von Infrarotgassensoren verwendet werden können.

Ferner ist aus US 2011/0147592 A1 eine Anordnung bekannt, die zur Spektralanalyse von kleinen Konzentrationen von Gas angepasst ist. Die Anordnung verwendet eine kleinvolumige Messzelle sowie Druckänderungen des Messgases.

Demgegenüber liegt der vorliegenden Erfindung die Aufgabe zugrunde, eine Anordnung und ein Verfahren zum Messen einer Gaskonzentration, insbesondere zur kapnometrischen Messung des Anteils von $CO_2$ in Atemluft, zur Verfügung zu stellen, die bei ausreichender Messgenauigkeit und Messgeschwindigkeit den Einsatz im mobilen Bereich und im Notfallbereich verbessert.

Diese Aufgabe wird durch eine Anordnung nach Anspruch 1 gelöst.

Im Rahmen der erfindungsgemäßen Anordnung handelt es sich hierbei um quasi-punktförmige Lichtquellen. Aufgrund ihrer geringen thermischen Masse ist eine hohe Messrate erzielbar, die Start-up Zeit wird signifikant gegenüber großflächigen thermischen Strahlen reduziert und es ist sehr wenig Leistung zur Erzielung hoher Glühwendeltemperaturen notwendig. Durch die geringe notwendige Leistung wird die Akkulaufzeit eines entsprechenden Messgeräts, insbesondere eines Kapnometers, signifikant gesteigert werden. Dies alles sorgt dafür, dass eine entsprechende Anordnung für den mobilen Einsatz und in der Notfallmedizin optimal geeignet ist. Da die Emission in einem sehr kleinen geometrischen Gebiet erfolgt, ist außerdem der Verlust an ausgestrahltem Licht sehr gering, sodass eine sehr hohe Lichtausbeute auf den verwendeten Detektoren erzielt wird, was ebenfalls den Energieverbrauch zusätzlich reduziert. So kann ein kleiner und leichter Sensor mit hoher Akkulaufzeit realisiert werden. Weitere Vorteile bestehen darin, dass keine mechanisch bewegten Bauteile im optischen Strahlengang verwendet werden, sodass eine robuste und kostengünstige Messanordnung verwirklicht werden kann.

Die Grundidee der vorliegenden Erfindung besteht darin, dass eine kleine punktförmige bzw. quasi-punktförmige thermische Lichtquelle bei vergleichsweise hoher Temperatur und einer verlustarmen optischen Abbildung der Quelle auf kleine, insbesondere quasi-punktförmige, Detektoren, beispielsweise Fotodioden, erfolgt. Der Emissionsbereich ist vorzugsweise deutlich kleiner als 1 mm$^2$. Bei Mikroglühfadenlampen kann eine Glühwendel bzw. ein Glühfaden innerhalb einer Fläche von beispielsweise 0,2 mm x 0,5 mm gewendelt sein, also eine Fläche von 0,1 mm$^2$ abdecken. Eine solch kleine Wendel ist in einem evakuierten oder mit einer Schutzatmosphäre gefüllten Glaskolben angeordnet bzw. eingekapselt, was die Wendel vor Korrosion schützt. Die Kapselung ist bei den gegebenenfalls eingesetzten höheren Temperaturen notwendig. Hierdurch wird eine hohe Temperatur des thermischen Strahlers möglich und daher auch eine hohe Lichtintensität auch im MIR-Bereich. Dies ermöglicht ein gutes Signal-Rausch-Verhältnis. Durch die Anordnung im evakuierten Glaskolben ergibt sich auch ein geringerer Wärmeaustausch des Strahlers mit der Umgebung und daher weniger Verlustleistung sowie ein mechanischer Schutz und eine Halterung, was für die Fertigung günstig ist.

Zur Minimierung von Leistungsverlusten durch eine zu große Ausdehnung der Lichtquelle ist vorteilhafterweise vorgesehen, dass die Kapsel der Mikro-Glühfadenlampe einen Durchmesser von weniger als 2 mm, insbesondere weniger als 1,5 mm, insbesondere weniger als 1 mm, aufweist. Zusätzlich oder alternativ hierzu beträgt vorteilhafterweise ein größter linearer Abstand zwischen zwei Punkten der Wendel weniger als 1 mm, insbesondere weniger als 0,5 mm. Bei geschickter Auswahl der Wendelgeometrie lässt sich so eine sehr hohe ausgesandte Leistung in sehr geringem Gesamtvolumen der Wendel erreichen. Ferner ist es zusätzlich oder alternativ vorteilhaft, wenn eine Einhüllende der Wendel in einer Projektionsrichtung, in der die Einhüllende eine maximale Einhüllenden-Projektionsfläche annimmt, eine maximale Einhüllenden-Projektionsfläche von weniger als 0,1 mm$^2$, insbesondere weniger als 0,02 mm$^2$, aufweist. Auch dieses Maß, welches die Fläche in einer Projektion aus einer Blickrichtung betrifft, in der die Projektion der Wendel auf die zur Blickrichtung senkrechte Ebene maximal ist, sorgt für eine vorteilhafte Kompaktheit der lichterzeugenden Wendel.

Erfindungsgemäß ist der Sensor oder sind die Sensoren als infrarotempfindliche Photodioden ausgebildet, dessen oder deren sensitive Fläche jeweils kleiner als 1 mm$^2$, insbesondere kleiner als 0,15 mm$^2$, ist. Solche kleinflächigen Fotodioden haben wenig Last von thermischem Rauschen. Weiterhin lässt sich das von der quasi-punktförmigen Lichtquelle ausgesendete Licht durch optische Komponenten sehr gut auf die kleinflächigen Infrarotsensoren bündeln. Hierdurch wird ein gutes Signal-Rausch-Verhältnis erzielt. Hierbei handelt es sich ferner um kostengünstige und robuste Bauteile, sodass auch in Bezug auf die Sensorik eine kostengünstige und robuste Lösung zur Verfügung steht.

Typische brauchbare Photodioden haben eine geringe Detektorfläche, beispielsweise ca. 0,35 mm x 0,35 mm, also eine Fläche von 0,1225 mm$^2$, was in der Größenordnung der strahlenden Fläche der erfindungsgemäß verwendeten Lichtquelle ist. Hier kann also eine optische Punktabbildung realisiert werden. Die kleinen Detektoren haben eine hohe Grenzfrequenz, sodass die benötigte Messrate ohne Probleme realisiert werden kann. Im Vergleich zu Thermopiles und pyroelektrischen Detektoren haben kleine Fotodioden eine begrenzte spektrale Empfindlichkeit und sind somit weniger empfindlich gegenüber thermischen Störstrahlungsquellen.

In einer vorteilhaften Weiterbildung ist eine Steuerungsvorrichtung umfasst, die zur leistungsgeregelten Ansteuerung und/oder Modulation der Mikro-Glühfadenlampe ausgebildet ist, wobei die Steuerungsvorrichtung, um einen Ist-Wert der abgegebenen Leistung zu bestimmen, zur Bildung eines Produktes aus an der Mikro-Glühfadenlampe gemessenem Strom und gemessener Spannung ausgebildet ist und/oder mit einer bei der Mikro-Glühfadenlampe angeordneten Photodiode signalverbunden ist, die einen Teil des von der Mikro-Glühfadenlampe erzeugten Lichts empfängt. Obwohl viele Filamentquellen üblicherweise über ihre Spannung angesteuert werden, ist aufgrund der Temperaturabhängigkeit ihres Innenwiderstandes eine Spannungssteuerung weniger geeignet, eine konstante und reproduzierbare Leistungsabgabe anzustellen als eine Leistungssteuerung. Die Stromaufnahme und damit einhergehend die aufgenommene elektrische Leistung sowie folglich auch die abgegebene optische Leistung sind somit von der Temperatur der Glühwendel abhängig. Dies zeigt sich insbesondere beim "Einlaufen" der Quelle nach einem Kaltstart und bei Änderungen des Arbeitspunktes deutlich. Somit ist für einen sogenannten AC-Betrieb eine Leistungsansteuerung der Filamentquelle sinnvoll.

Zur Leistungsregelung ist möglich, Strom und Spannung der Strahlungsquelle elektronisch zu erfassen und zu multiplizieren und dieses Produkt als Ist-Größe für eine Regelung zu nutzen. Eine besonders schnelle Regelung der Leistung der Lichtquelle, die für die Modulation der Lichtquelle günstig ist, kann erreicht werden, indem Strom und Spannung analog erfasst und analog multipliziert werden. So kann dann auch ein rein analoger Regler eingesetzt werden, was Vorteile in der Geschwindigkeit und in den niedrigen Kosten hat. Alternativ ist eine digitale Umsetzung der Produktbildung und Regelung möglich.

In einer alternativen Implementierung einer Leistungsregelung wird ein Teil der emittierten Strahlungsleistung möglichst frei von optischen Störquellen mittels eines Sensors, beispielsweise einer Photodiode, erfasst, vorzugsweise einer rauscharmen, kostengünstigen VIS- oder NIR-Photodiode. Der in der Photodiode generierte Photostrom ist direkt proportional zur erfassten optischen Leistung. Somit kann das verstärkte Signal des Photostroms als Istwert für die Regelung der optischen Leistung genutzt werden. Diese Lösung erfordert eine möglichst vollständige Abschottung von Fremdlicht. Ferner ist sicherzustellen, dass ein repräsentativer Anteil des emittierten Lichtes erfasst wird und für die Regelung genutzt wird.

In einer bevorzugten Anordnung wird das IR-Licht gebündelt durch die Messzelle geleitet und nach Durchgang durch die Messzelle mittels eines spektral neutralen optischen planparallelen oder gewölbten Transmissions- oder Reflexionsgitters auf zwei oder mehr Sensoren aufgeteilt, wobei das Transmissionsgitter oder Reflexionsgitter insbesondere eine Gitterkonstante aufweist, die um einen Faktor von 30 oder mehr, insbesondere um einen Faktor von 50 und mehr, kleiner ist als ein Durchmesser eines Lichtflecks auf dem Transmissions- oder Reflexionsgitter. Unter dem Lichtfleck wird in diesem Fall der Lichtfleck verstanden, der von dem Licht erzeugt wird, das von der Mikro-Glühfadenlampe abgestrahlt und durch den optischen Pfad bis zum Gitter gelangt. Eine Gitterkonstante von 300 $\mu$m oder weniger, insbesondere von 50 $\mu$m oder weniger, ist dabei vorteilhaft. Mit dieser Aufteilung durch ein spektral neutrales Gitter wird sichergestellt, dass das gesamte Licht sowohl der Absorptionswellenlänge als auch der Referenzwellenlänge bis zu der Aufteilung denselben optischen Pfad durchläuft und somit keine systematischen Unterschiede in der Absorption erleidet, abgesehen von der zu messenden Absorption im Messgas. Mittels des spektral neutralen Gitters wird das gesamte Licht, dass die Messzelle durchquert hat, unabhängig von der jeweiligen Wellenlänge in zwei oder mehr räumlich voneinander getrennte Pfade aufgeteilt und auf voneinander getrennte Detektoren bzw. Detektoreinheiten geleitet. Jede Detektoreinheit kann beispielsweise einen Detektor und ein optisches Bandpassfilter aufweisen. Diese sollten unterschiedliche spektrale Empfindlichkeiten aufweisen, so dass das empfangene Licht auf dem einen Detektor stark von der Absorption der Messgröße, beispielsweise $CO_2$, abhängig ist und das auf dem anderen Detektor, dem Referenzdetektor, empfangene Licht hiervon möglichst unabhängig ist.

Weiterhin sollten die spektralen Empfindlichkeitsbereiche der Detektoreinheiten möglichst nahe beieinanderliegen, damit Auswirkungen spektral abhängiger, also dispersiver, breitbandiger Störungen, wie etwa die temperaturabhängige optische Dispersion oder die durch Temperaturveränderung hervorgerufene spektrale Änderung der Emissionscharakteristik einer thermischen Quelle, sich nicht oder nur kaum unterschiedlich auf die detektierten Signale auswirken. Störungen der Transmission im optischen Pfad, die gegenüber dem Abstand der beiden Empfindlichkeitsbereiche der Filter breitbandig sind, breitbandiges Fremdlicht sowie Intensitätsschwankungen der Lichtquelle können durch Verrechnung der empfangenen Detektorsignale gegenüber dem Absorptionsmesssignal unterdrückt werden. So wird die Genauigkeit der Messung der Zielgröße, beispielsweise die Konzentration von $CO_2$, deutlich erhöht.

Es ist hierbei vorteilhaft, wenn die mit den Detektoreinheiten erfassten Lichtanteile möglichst demselben Ursprungsort der Lichtquelle entstammen und möglichst denselben optischen Pfad von der Quelle bis zur Aufteilung auf die Detektoreinheiten gefolgt sind. Dies ist bei einer erfindungsgemäß kleinflächigen Filamentquelle in der Regel in hohem Maße gegeben. Weiterhin sollte die Aufteilung des Lichtes möglichst gleichverteilt und ohne eine starke räumliche Abhängigkeit erfolgen, da sonst die detektierten Lichtanteile von unterschiedlichen Lichtpfaden herrühren. Somit wirken die Quell- und Pfadstörungen auf gleiche Weise auf die empfangenen Lichtsignale und können bei der Ermittlung der Messgröße kompensiert werden.

Unter einem spektral neutral wirkenden Gitter wird im Rahmen der vorliegenden Erfindung eine gleichverteilte, fein gegliederte Strukturierung eines optischen Bauteils im Strahlengang verstanden, welches die Richtung des eintreffenden Lichts in zwei oder mehr Richtungen zu entsprechenden Detektoreinheiten ablenkt. Durch diese fein gegliederte und innerhalb der Empfangsapertur des optischen Bauteils gleichmäßig verteilte Struktur erfolgt eine diversifizierte Aufteilung des Lichtes auf die Detektoreinheiten. Hierdurch wird erreicht, dass das Lichtbündel in viele kleine Unterbündel zerlegt wird, welche dann auf die Detektoren aufgeteilt und hier gebündelt erfasst werden. Störungen, deren räumliche Ausdehnung größer als die eines Unterbündels ist, wirken auf mehrere Unterbündel und somit stochastisch auf die mehreren Detektoreinheiten in gleicher Weise und können auf diese Weise kompensiert werden. Kleinere Störungen hingegen wirken im Extremfall selektiv auf nur einen Detektorpfad. Der hierdurch betroffene Signalanteil eines einzelnen Unterbündels und der darin enthaltenen kleinen Störungen ist gegenüber dem Gesamtsignal jedoch sehr klein, so dass der auf diese Weise entstehende Fehler im Messsignal vernachlässigt werden kann.

Die Struktur eines solchen Gitters sollte möglichst klein gegenüber der Eintrittsfläche des Gesamtlichtbündels, also den Lichtfleck, sein, damit der Intensitätsanteil eines einzigen Unterbündels gegenüber der einfallenden Gesamtintensität möglichst gering ist. Die Struktur soll andererseits groß genug sein, damit wellenlängenabhängige Beugungseffekte nicht die Lichtaufteilung dominieren. Bei einer Wellenlänge im mittleren Infrarotbereich von zwischen 4 und 5 μm ergibt sich sinnvollerweise eine Gitterkonstante von zwischen 20 und 300 μm, vorzugsweise 200 μm oder weniger, weiter vorzugsweise 80 μm oder weniger. Die Untergrenze liegt bei ca. 20 μm, damit die Beugungseffekte nicht zu groß werden. Zusätzlich sollten die Umlenkwinkel nicht im Bereich der Beugungsmaxima der genutzten Wellenlängen liegen. Für eine gute Störunterdrückung sollten die Flächenanteile der mehreren Richtungen möglichst gleich groß sein und somit die Lichtintensitäten gleichmäßig auf die mehreren resultierenden Lichtpfade aufgeteilt sein. Die Querschnittsfläche der Unterbündel sollten die gleiche Größe aufweisen, damit einwirkende Störungen im Strahlengang auf die Unterbündel in gleicher Weise wirken und so vollständig kompensiert werden können.

Ein entsprechendes spektral neutrales Gitter kann als Reflexionsgitter ausgeführt sein. In diesem Fall wird das Licht aufgeteilt und zusätzlich umgelenkt, beispielsweise um 90°. Hierdurch wird eine sehr kompakte Sensorbauform möglich. Das Gitter kann durch eine zwei- oder dreidimensionale Form, die in der dritten Raumrichtung kontinuierlich erweitert ist, gegeben sein, beispielsweise in Form einer Sägezahnstruktur in Form einer Satteldachstruktur oder komplexere Formen, wie eine Pyramidenstruktur. Hierdurch sind zwei oder mehrere Richtungen der Lichtaufteilung möglich. Auf diese Weise kann eine gute räumliche Aufteilung und Trennung des Lichts auf zwei, drei oder mehr Detektoren erreicht werden. Durch eine zusätzliche Wölbung, beispielsweise in Form einer Paraboloiden, des gesamten Gitters kann gleichzeitig eine Fokussierung des einfallenden Lichtes auf die Empfänger bewegt werden, ohne dass weitere optische Bauelemente, beispielsweise Hohlspiegel an den Empfängern, hierfür erforderlich sind.

Alternativ kann das Gitter ein Transmissionsgitter sein, welches auf oder in einem planparallelen, im Wellenlängenbereich der Messung, insbesondere im IR-Bereich, transparenten Material, insbesondere Glas, ausgeführt ist. Im Gegensatz zum Reflexionsgitter bewirkt hierbei ein Brechzahlunterschied des Substrates zur Umgebung bzw. innerhalb des Substrates eine Änderung und Aufteilung der Richtung des einfallenden Lichtes. Auch hier ist es sinnvoll, zweidimensionale oder dreidimensionale Strukturen zu verwenden, die eine zusätzliche Fokussierung ermöglichen.

Die Kombination der Gitterstruktur mit fokussierenden Elementen oder Strukturen zeichnet sich durch eine einfache Positionierung der optischen Bauteile im Strahlengang bei der Sensorfertigung aus. Dies betrifft insbesondere die Transmissionsanordnung.

Für die Anwendung im genannten mittleren Infrarotbereich ist beispielsweise eine Neigung von Gitterschrägen eines Reflexionsgitters von etwa 27° günstig, welche einen Winkel zwischen den Detektoren von ca. 100° bei einer exakten Umlenkung um 90° erzielt, ohne dabei eine Abschottung von Lichtanteilen durch das Gitter selbst zu verursachen. Durch diese Aufspreizung der resultierenden Lichtstrahlen von ca. 100° ist es möglich, die Detektoreinheiten in der Nähe des Gitters zu positionieren, wodurch ein sehr kompakter Aufbau realisiert wird. Die Umlenkung des auf das Gitter einfallenden Lichts um genau 90° ist für die Justage und Positionierung der Detektoreinheiten und somit für die Fertigung sehr günstig.

Vorzugsweise ist die Messzelle als innen diffus oder hochglänzend verspiegelte Röhre ausgebildet, an deren einem Ende die Mikro-Glühfadenlampe und an deren anderem Ende der Sensor oder die Sensoren mit den vorgeschalteten Bandpassfiltern angeordnet sind.

Dies ermöglicht eine vollständige Transmission des emittierten Lichts und erhöht somit die Messgenauigkeit. Eine diffuse Verspiegelung, beispielsweise durch eine Mikrostrukturierung der spiegelnden Oberfläche, bewirkt eine zumindest teilweise Entkopplung des bei den Empfängern ankommenden Lichts vom Ursprungsort und somit eine Verminderung systematischer Fehler. Eine hochglänzende Verspiegelung weist den Vorteil geringerer Verluste an Lichtintensität auf. Ein beispielhaftes geeignetes Material für eine Innenverspiegelung ist Gold, welches im IR-Bereich eine gute Reflektivität aufweist und aufgrund seiner Eigenschaft als Edelmetall wenig anfällig gegenüber Korrosion ist.

In einer Alternative der Erfindung ist der wenigstens eine Bandpassfilter als Doppel-Bandpassfilter ausgebildet, der IR-Licht sowohl im Messwellenlängenbereich als auch im Referenzwellenlängenbereich durchlässt, wobei der Doppel-Bandpassfilter einem einzelnen Sensor vorgeschaltet ist. In einer vorteilhaften Weiterbildung der Anordnung ist die Steuerungsvorrichtung ausgebildet und eingerichtet, die Mikro-Glühfadenlampe zwischen einem Arbeitspunkt niedriger Leistung und einem Arbeitspunkt hoher Leistung zu modulieren, in denen das jeweilige Emissionsspektrum unterschiedliche Anteilsverhältnisse im Messwellenlängenbereich und im Referenzwellenlängenbereich aufweist.

Diese Doppel-Bandpass-Anordnung beruht auf dem Prinzip, dass die Filterbereiche für die Gasabsorption und die Referenzwellenlänge in einem optischen Doppelbandpassfilter zusammengefasst werden. Das Licht wird in allen Bereichen geblockt, außer in zwei Durchlassbereichen bei der Gasabsorption und der Referenzwellenlänge. Dieser Filter befindet sich im optischen Pfad zwischen Lichtquelle und Empfänger. Die vorzugsweise leistungsgeregelte Quasi-Punktlichtquelle wird abwechselnd in einem Arbeitspunkt mit niedriger Leistung bzw. niedriger Temperatur betrieben und in einem Arbeitspunkt mit hoher Leistung. In dem Arbeitspunkt mit niedriger Leistung überstreicht das Emissionsspektrum vor allem den Bereich der Gasabsorption. In dem Arbeitspunkt mit hoher Leistung gelangt durch beide Filterbereiche Licht auf den Empfänger. Das detektierte Licht ist somit zu einem Zeitpunkt durch die Absorption des Zielgases dominiert und im nächsten Zeitpunkt ist ein zusätzlicher Anteil enthalten, der die Lichttransmission durch die Zelle beinhaltet. Dies beinhaltet somit die Referenzmessung. Ist die Änderung der Gaskonzentration innerhalb dieses Zeitintervalls vernachlässigbar gering, so kann anhand der Messwerte zu den Zeitpunkten bei hoher und niedriger Temperatur bzw. Leistung die Konzentration der Zielgröße bestimmt werden. Dabei werden wie in einer Zweidetektoranordnung breitbandige Transmissionsänderungen des optischen Pfades oder durch andere Absorber unterdrückt. In dieser Anordnung ist nur ein Empfänger erforderlich.

Die Signalverarbeitung und -auswertung für diesen Fall sei im Folgenden kurz beschrieben. Für eine vereinfachte Beschreibung wird angenommen, dass der Lichtanteil des Referenzbereichs im ersten Arbeitspunkt vernachlässigbar ist. Dann gilt für die am Detektor im Arbeitspunkt 1 (AP1) ankommende Lichtintensität $I_{AP1}$:

$$I_{AP1} = k_S I_{01} e^{-\alpha xc}, \qquad (1)$$

wobei $k_s$ ein spektral unabhängiger Dämpfungsfaktor ist, der Störungen und Konstanten abbildet, die beispielsweise die Verstärkung, die optische Abbildung im System und andere Einflüsse beschreiben. Der Faktor $\alpha$ im Exponenten bezeichnet den wellenlängenabhängigen Absorptionskoeffizienten des Messgases. Die Terme $x$ und c bezeichnen die Messstrecke in der Messzelle und die Konzentration des Messgases.

Im zweiten Arbeitspunkt (AP2) gilt für die am Detektor ankommende Lichtintensität $I_{AP1}$:

$$I_{AP2} = k_S I_{01}(k_a e^{-\alpha xc} + k_b). \qquad (2)$$

Hier beinhalten die Faktoren $k_a$, $k_b$ die Änderung der Lichtintensität der Quelle im Filterbereich des Absorptionssignals beim Übergang vom Arbeitspunkt 1 zum Arbeitspunkt 2 bzw. das Verhältnis der Lichtintensität der Quelle im Filterbereich des Absorptionssignals beim Arbeitspunkt 1 zu der Lichtintensität der Quelle im Filterbereich des Referenzsignals beim Arbeitspunkt 2. Diese Faktoren enthalten somit auch die Änderung der Intensität der Lichtquelle in den beiden Fenstern beim Übergang vom Arbeitspunkt 1 zum Arbeitspunkt 2. Nach Umformung ergibt sich für die Konzentration bzw. die Zielgröße c:

$$c = k_S I_{01} \left( \frac{\frac{I_{AP2}}{I_{AP1}} - k_a}{k_b} \right) \frac{1}{-\alpha x}. \qquad (3)$$

dieses Messergebnis ist stark von den Verhältnissen der Intensitäten der Lichtquelle im Arbeitspunkt 1 und im Arbeitspunkt 2 abhängig. Die praktische Auswertung erfolgt vorzugsweise anhand von kalibrierten Kurven oder look-up-Tabellen, da $k_a$, $k_b$ im praktischen System zur Laufzeit nicht bestimmt werden können.

Die Doppelbandpassfilter-Anordnung erfordert nur einen optischen Kanal und eine Detektoreinheit, sodass sich ein robuster und optisch einfacher Aufbau ergibt, der gegenüber optischen Störungen, Veränderungen der Verstärkung und der Empfindlichkeit des Empfängers eine hohe Sicherheit bietet. Die zur Verfügung gestellte optische Leistung wird nicht auf mehrere Empfänger aufgeteilt. Die Modulation der Quasi-Punktlichtquelle kann durch eine optische oder rein elektronische Leistungsregelung präzise erfolgen, wodurch die Referenzierung bzw. Referenzmessung sehr stabil wird. Anhand des zeitlichen Verlaufs des detektierten Signals während der Modulation können die Arbeitspunkte überprüft werden. Weitere Vorteile dieser Anordnung sind die geringe Leistungsaufnahme aufgrund der Verwendung nur eines Empfangskanals und die geringen Fertigungskosten aufgrund der geringen Anzahl von optischen und elektronischen Bauteilen.

In einer weiteren vorteilhaften Weiterbildung der vorgenannten Anordnungen sind Mittel umfasst, die zu einer temporären Druckerhöhung und/oder Druckabsenkung des Gasgemischs in der Messzelle ausgebildet sind, insbesondere eine Pumpe und/oder ein oder mehrere, insbesondere schaltbare, Ventile. Solche Ventile können schaltbare Ventile oder andere Ventile, wie Rückschlagventile oder Druckbegrenzungsventile sein. Vorzugsweise ist eine Steuervorrichtung umfasst, die ausgebildet ist, die Mittel anzusteuern, um temporär einen Gasauslass der Messzelle zu blockieren und/oder die Menge an zu analysierendem Gasgemisch in der Messzelle zu erhöhen und/oder abzusenken. Alternativ kann die Druckvariation auch mit Hilfe eines externen Geräts zusätzlich zum eigentlichen Sensormodul genutzt werden.

Diese Maßnahme stellt vorteilhafterweise eine Ergänzung zu Sensoren auf Basis herkömmlicher Seitenstrommessanordnungen für die Sensorkalibrierung dar, die in größeren Zeitintervallen sinnvoll ist. Es wird eine signifikante Druckänderung zur Referenzierung verwendet, zum Beispiel durch eine deutliche Erhöhung des Drucks oder auch eine Variation des Drucks von einem Unterdruck bis zu einem Überdruck in der Messzelle. Zur Erzeugung eines Überdrucks kann beispielsweise eine Pumpe in Kombination mit einem Rückschlagventil vor der Messzelle verwendet werden, wobei die Strömungsrichtung geändert wird und die Pumpe, die anderenfalls Gas aus der Messzelle absaugt, eine Komprimierung des Gases in der Messzelle bewirkt. Es kann gegebenenfalls wirtschaftlich sinnvoll sein, ein externes Gerät zu benutzen, welches die erforderliche Hardware umfasst, um die Druckmanipulation durchzuführen. Ein derartiges Zusatzgerät wird vorteilhafterweise an die pneumatischen Eingänge und Ausgänge der Messzelle angeschlossen. Wenn das Sensorsystem bzw. die Anordnung für eine derartige externe Kalibrierung ausgelegt ist, können die Rohdaten abgegriffen werden, Kalibrierdaten erfasst werden und aktualisierte Kalibrationsparameter auf den Sensor übertragen werden. Auf diese Weise ist es möglich, eine zyklisch erforderliche Kalibrierung ohne aufwändige Kalibriergase kostengünstig zu gestalten.

Diese Maßnahme findet vornehmlich im Seitenstrom statt, da das im Seitenstrom abgesaugte Gasgemisch vergleichsweise einfach gestaut und druckmanipuliert werden kann. Hiermit wird eine über das Absorptionsgesetz hinausgehende Nichtlinearität der Absorption der Zielgröße genutzt, um sie von schwächeren und vor allem breitbandigen Hintergrundabsorptionen zu trennen und so eine selektive und robuste Messung der Zielsubstanz zu ermöglichen. Auch eine Intensitätsdrift der Quelle und Änderungen der Transmission der Übertragungsstrecke werden bei diesem Verfahren in Bezug auf die Zielgröße unterdrückt. Da das Aufstauen des Messgases eine Unterbrechung der eigentlichen Messung bedeutet, handelt es sich um ein zyklisches Verfahren. Es ist mit einer kontinuierlichen einkanaligen Messung ebenso kombinierbar wie mit einer Mehrkanalmessung, um unter anderem Störeffekte wirkungsvoll zu kompensieren.

Die Nichtlinearitätsmessung ist nicht auf den Anwendungsfall der Absorptionsmessung mit kleinvolumigen IR-Lichtquellen begrenzt. Im vorliegenden Fall kann sie zu einer zuvor beschriebenen kontinuierlichen Messung einer Zielgröße und Konzentrationsbestimmung hinzutreten. Die Bestimmung der Nichtlinearität setzt voraus, dass über den Zeitraum des Druckaufbaus bzw. der Druckänderung mehrere Messungen erfolgen, so dass aus dem Zusammenhang zwischen Messwerten und Druckentwicklung auf die Nichtlinearität geschlossen werden kann. Im Rahmen der Nichtlinearitätsmessung wird dazu zyklisch, vorzugsweise bei einer hohen Konzentration der Zielgröße, beispielsweise bei expiratorischem $CO_2$, der Fluss des Messgases kurzzeitig aufgestaut. Hierfür kann beispielsweise ein Absaugen des zu messenden Gases für kurze Zeit gestoppt werden, indem beispielsweise die Flussrichtung einer Pumpe umgekehrt wird, wobei die Pumpe gegen ein Ventil, insbesondere ein Rückschlagventil, vor der Messzelle arbeitet, und so das Gasgemisch in der Zelle verdichtet. Gegebenenfalls ist eine Absenkung des Drucks in analoger Weise möglich, indem das Ventil vor der Messzelle gedrosselt wird und die Flussrichtung der Pumpe beibehalten wird. Auf diese Weise wird die Transmission der Anordnung bei unterschiedlichen Drücken des Gases in der Messzelle erfasst. Ist in der Zelle eine signifikante Druckänderung erreicht, so beginnt die Pumpe wieder mit dem Absaugen und der kontinuierlichen Messung.

Die Änderung des Druckes hat keinen Einfluss auf die Lichtquelle, die Lichtübertragung und den Empfänger. Diese Größen können während der Dauer der Druckänderung als konstant angenommen werden. Signifikante Änderungen, Drifterscheinungen, Alterseffekte etc. haben eine größere Zeitkonstante als die Dauer der Druckänderung und Nichtlinearitätsmessung. Im Gegensatz zu der optischen und elektronischen Übertragung ändert sich aber die Absorption in der Messzelle mit dem Druck. Da ebenfalls der Druck des Gases in der Messzelle gemessen wird, können die unbekannten Übertragungsdaten des Messpfades auf diese Weise bestimmt werden.

Durch eine geeignete Auslegung des optischen Filters kann im Zusammenhang mit der optischen Gasabsorption der Zielgröße die Stärke der Nichtlinearität der Absorption der Zielgröße beeinflusst werden. Eine derartige Nichtlinearität der Absorption tritt bei anderen sich überlagernden breitbandigen und in der Regel schwächeren Absorbern, welche ebenfalls im Gasgemisch enthalten sein können, nicht auf. Anhand dieser Eigenschaft kann die Zielgröße auch ohne die Verwendung einer zusätzlichen Referenzwellenlängenmessung selektiv bestimmt werden. Der auf diese Weise ermittelte Konzentrationswert der Zielgröße kann zur Korrektur der Werte zwischen den Druckzyklen genutzt werden.

Physikalisch ergibt sich die Nichtlinearität aus der Überlagerung des wellenlängenabhängigen Transmissionsverlaufs des Filters mit dem wellenlängenabhängigen Absorptionskoeffizienten des Messgases, die der Transmission des Lichts durch die Messzelle und das zu messende Gas im Wellenlängenbereich des optischen Filters und der Absorption des Messgases zugrunde liegt. Wenn das Transmissionsfenster des Filters die Ausläufer der Absorptionsbande des Messgases umfasst, wird bei einer Konzentrationserhöhung des Messgases in der Messzelle die Absorption des durchstrahlenden Lichts nichtlinear mit der Erhöhung der Konzentration zunehmen. Dies ist ein Effekt, der ausschließlich auf das Messgas zurückzuführen ist, sofern andere Gase in dem Gasgemisch und andere Effekte der Messvorrichtung eine Lichtschwächung bewirken, die innerhalb des entsprechenden spektralen Fensters des Filters weitgehend unabhängig von der Wellenlänge ist.

Es wird nur ein Übertragungskanal von einer Lichtquelle auf eine Detektoreinheit benötigt, wobei eine Detektoreinheit als Detektor und gegebenenfalls optischer Filter zu verstehen ist. Das Licht der Quelle sollte amplitudenmoduliert sein, da Gleichanteile zu unterdrücken sind. Es werden im Folgenden die Wechselsignalamplituden betrachtet. Die detektierte Lichtleistung auf der Detektorfläche bzw. die am Detektor empfangene Lichtintensität $I_M$ kann beschrieben werden als

$$I_M = I_0 k_s T_{ges}, \qquad\qquad (4)$$

wobei $I_0$ die von der Lichtquelle abgegebene Lichtintensität ist, $k_S$ ein spektral unabhängiger Dämpfungsfaktor, der Störungen und Konstanten umfasst, welche beispielsweise die Verstärkung, Abbildung etc. im System beschreiben, und $T_{ges}$ die wellenlängenabhängige Transmission des Gesamtsystems beschreibt. Dieser Faktor ist ein Produkt

$$T_{ges} = T_G T_S T_F \qquad (5)$$

aus der wellenlängenabhängigen und druckabhängigen Transmission $T_G$ des Zielgases, der wellenlängenunabhängigen und druckabhängigen Transmission $T_S$ störender Gase sowie der druckunabhängigen wellenlängenabhängigen Transmission $T_F$ des optischen Systems aus optischem Filter, spektraler Empfindlichkeit des Detektors, dem spektralen Verlauf der Emissionsquelle, sowie Übertragungseigenschaften der Zelle. Für das Verfahren ist es wichtig, dass der Transmissionsbereich des Bandpassfilters nicht nur sehr schmalbandig auf das Maximum einer Absorptionslänge des Zielgases abgestimmt ist, sondern auch Bereiche mit niedrigerer Absorption umfasst. Weiterhin sollte der Filterdurchlassbereich so gewählt sein, dass störende Gase hier einen möglichst konstanten Absorptionsverlauf aufweisen. Außerdem ist die Filterbandbreite unter Berücksichtigung der angeführten Anforderungen schmal zu halten, damit spektrale bzw. dispersive, beispielsweise temperaturabhängige, Veränderungen der meist breitbandigen Lichtquellen und des Detektors sich nicht oder nur vernachlässigbar auf das Messergebnis auswirken.

Die Formel (4) kann unter Berücksichtigung der Druckabhängigkeit und der Wellenlängenabhängigkeit als Summe der $n$ Spektralanteile beschrieben werden:

$$I_M(p) = \frac{I_0}{n} k_S T_S(p) \sum_n T_{Gn}(p) T_{Fn}. \qquad (6)$$

Nach Anwendung des Absorptionsgesetzes und unter Berücksichtigung der Druckabhängigkeit der Teilchendichte im Messgas ergibt sich:

$$I_M(p) = \frac{I_0}{n} k_S \, e^{-\alpha_S x c_S \frac{p}{p_0}} \sum_n e^{-\alpha_{Gn} x c_G \frac{p}{p_0}} k_{Fn}, \qquad (7)$$

und nach Logarithmieren der Formel (7):

$$ln \, I_M(p) = ln \left( \frac{I_0}{n} k_S \right) - \alpha_S x c_S \frac{p}{p_0} + ln \left( \sum_n e^{-\alpha_{Gn} x c_G \frac{p}{p_0}} k_{Fn} \right). \qquad (8)$$

Der Logarithmus des Messsignals kann somit als Summe von drei Termen ausgedrückt werden, von denen einer druckunabhängig ist, einer vom Druck linear abhängig und ein Dritter einen nichtlinear vom Druck abhängigen Anteil enthält. Der linear abhängige Anteil enthält die Störgasabsorption, der nichtlineare Anteil die Zielgasinformation. Durch weitere Signalverarbeitung können diese Anteile voneinander getrennt werden, wodurch Störeinflüsse im Messsignal eliminiert werden.

Dies kann beispielsweise durch Ableiten der Funktion (8) nach dem Druck erfolgen, wodurch die druckunabhängigen Signalbestandteile aus dem Messsignal entfernt werden und als einziger druckabhängiger Bestandteil der nichtlineare Anteil übrig bleibt, der auf das Zielgas zurückgeht. Aufgrund der Linearität der Druckabhängigkeit des möglichen Störgasanteils verbleibt hiervon nach der Ableitung lediglich ein konstanter Wert:

$$\frac{d}{dp} ln \, I_M(p) = -\frac{\alpha_S x c_S}{p_0} + \frac{d}{dp} ln \left( \sum_n e^{-\alpha_{Gn} x c_G \frac{p}{p_0}} k_{Fn} \right). \qquad (9)$$

Damit bildet der Störgasanteil einen Offset und kann durch eine Betrachtung des Signalunterschieds bei verschiedenen Arbeitspunkten oder durch eine zweite Ableitung nach dem Druck vom Signalanteil des Zielgases getrennt werden. Der resultierende Signalverlauf hängt in nichtlinearer Weise, aber proportional zur Konzentration des Zielgases vom Druck ab. Der Verlauf dieses nichtlinearen Zusammenhangs, welcher vorteilhafterweise als funktionale Näherung oder als look-up-table zur Berechnung der Konzentration in der Signalauswertung hinterlegt werden kann, ist im Wesentlichen durch den Absorptionsverlauf des Zielgases und den gewählten Transmissionsverlauf des optischen Filters bestimmt und kann daher für bestimmte Konzentrationsmessbereiche optimiert werden. Eine Reihe von Störgrößen kann auf diese Weise unterdrückt werden, obwohl nur ein Messpfad im Aufbau genutzt wird. Durch rekursive Berechnung kann dann auch der Signalanteil des Störgases berechnet werden, was beispielsweise in der Kapnometrie einen näherungsweisen Rückschluss auf die Konzentration von $N_2O$ als stärkstem Störgas in der Kapnometrie zulässt.

Somit ist unter Verwendung nur eines Messpfades eine Konzentrationsbestimmung möglich, die, abgesehen vom SNR, unabhängig ist von der innerhalb der optischen Filterbandbreite wellenlängenstabilen Übertragung der Lichtintensität von der Quelle zum Detektor. Somit entfallen einige Fehlerquellen, die beispielsweise durch Alterung entstehen. Eine Null-Referenzmessung, zum Beispiel eine zyklische Umgebungsluftmessung, ist bei diesem Verfahren nicht notwendig. Stattdessen kann die absolute Konzentration bestimmt werden. Aufgrund des einfachen optischen Aufbaus ist eine hohe Lichtübertragung mit sehr geringen Verlusten und folglich sehr gutem SNR möglich. Es erfolgt eine effektive Unterdrückung anderer, bezogen auf die Filterbandbreite, breitbandiger Einflüsse, beispielsweise anderer Absorber. Entsprechend ist die Anordnung robuster gegenüber spektralen Schwankungen der Emission der Quelle und der Sensitivität des Detektors im Vergleich zu Verfahren mit zwei oder mehr getrennten Wellenlängenbereichen für die Messung der Gasabsorption und der spektralen Referenz. Dies rührt daher, dass bei der Referenzierung nicht der spektrale Abstand zwischen dem Absorptionsfilterbereich und dem Referenzbereich, sondern nur die Bandbreite des Filters im Absorptionsband wirksam wird. Auch in Kombination mit bestehenden Seitenstrommessverfahren kann dieses Verfahren zur Kompensation möglicher Drifteffekte integriert werden.

In analoger Weise kann anstelle der Modulation des Drucks in der Messzelle auch ein anderer Parameter aus den oben genannten Formeln gezielt verändert werden und die Änderung der Messung in Bezug auf ihre Nichtlinearität genutzt werden. Dies betrifft etwa den Absorptionskoeffizienten $\alpha$, die Absorptionslänge $x$ oder die Konzentration $c$.

Ebenfalls wird die der Erfindung zugrunde liegende Aufgabe gelöst durch ein Verfahren nach Anspruch 11.

Das erfindungsgemäße Verfahren verwirklicht damit die gleichen Vorteile, Merkmale und Eigenschaften wie die Erfindung gemäß der zuvor beschriebenen Anordnung. Das Verfahren, ebenso wie die Anordnung, sind vorteilhaft sowohl im Seitenstromverfahren als auch im Hauptstromverfahren einsetzbar.

Vorzugsweise wird die thermische Lichtquelle mit einer Messwiederholfrequenz $f_{Mess}$, die größer ist als 10 Hz, insbesondere größer als 25 Hz, moduliert, wobei eine Temperatur der Wendel während der Messung höher ist als 400 °C und einen Temperaturmodulationshub von wenigstens 300 °C, insbesondere wenigstens 500 °C aufweist, insbesondere im Maximum 1000 °C übersteigt. Die Messwiederholrate stellt sicher, dass auch bei einer schnellen Atemfrequenz eine ausreichende Abtastung des Verlaufs der $CO_2$-Konzentration in der Atemluft erfolgt. Hohe Atemfrequenzen liegen bei medizinischen Notfällen ebenso vor wie in der Neonatalmedizin, also bei Neu- und Frühgeborenen.

Die verwendeten hohen Temperaturen der thermischen Lichtquelle stellen sicher, dass eine ausreichende Intensität bei einer Wellenlänge von ca. 4,26 μm, also im Absorptionsband von $CO_2$ vorhanden ist. Die Temperatur liegt im Maximum allerdings deutlich höher als dies bei Kapnometrie üblich ist, wodurch deutlich höhere Intensitäten bei den Messwellenlängen zur Verfügung stehen. Dies wird ermöglicht durch die Auswahl einer quasi-punktförmigen Filamentquelle, die ohne wesentlichen Verlust an Lichtintensität in einem dünnen Glasgehäuse eingekapselt sein kann.

Die Mikro-Glühfadenlampe wird vorzugsweise leistungsgeregelt betrieben. Speziell bei den quasi-punktförmigen Filamentquellen der vorliegenden Erfindung, um die es sich bei den Mikro-Glühfadenlampen handelt, lässt sich auf diese Weise eine sehr genaue und reproduzierbare Regelung der Lichtintensität und des abgegebenen Spektrums des Lichts entsprechend der Temperatur der Glühwendel verwirklichen. Da die sehr kleine Glühwendel eine sehr geringe thermische Masse hat, sind Hystereseeffekte wie ein Temperaturnachlauf gegenüber der variabel eingespeisten Leistung auch bei den benötigten Modulationsfrequenzen akzeptabel. Ferner ist aufgrund der geringen thermischen Masse eine vergleichsweise schnelle Regelung und Temperaturänderung der Glühwendel möglich.

Wenn im Laufe einer Messung in zeitlichen Abständen hintereinander der Druck des Gasgemischs in der Messzelle erhöht und/oder abgesenkt wird und die Absorption in Abhängigkeit vom Druck gemessen wird, wobei zur Druckänderung insbesondere ein Abfluss des Gasgemischs unterbrochen und/oder ein Zufluss oder ein Abfluss des Gasgemischs durch eine Pumpe unterstützt und erhöht wird, so ist eine effiziente Art der quasi-kontinuierlichen Störunterdrückung und Konzentrationsbestimmung gegeben. Die Abhängigkeit der Absorption im Gasgemisch ist aufgrund der Abstimmung des Bandpasses, der den Absorptionsbereich des zu messenden Gases durchlässt, an das Absorptionsband des zu messenden Gases nichtlinear. Diese Nichtlinearität der Absorption in Abhängigkeit des Drucks in der Messzelle wird dazu verwendet, den Beitrag des zu messenden Gases von weiteren Beiträgen zu isolieren, die keinen entsprechenden nichtlinearen Beitrag liefern. Die Nichtlinearität der Druckabhängigkeit der gemessenen Absorption bietet somit eine unabhängige Referenzierung der Absorptionsmessung.

Vorteilhaft wird eine druckabhängige Messreihe in Bezug auf linear und nichtlinear vom Druck abhängige Anteile des Logarithmus des Messsignals analysiert und der nichtlinear vom Druck abhängige Anteil zur Messung der Gaskonzentration des zu messenden Gases oder zur Korrektur und/oder Kalibration einer Messung der Gaskonzentration des zu messenden Gases verwendet.

Die Grundlagen dieser Nichtlinearitätsmessung, der alternativ auch gezielte Änderungen der Absorptionskonstanten oder der Absorptionslänge zugrunde liegen können, sind oben im Zusammenhang mit der Anordnung bereits beschrieben worden, anhand der oben zitierten Formeln (4) bis (9). Diese Nichtlinearitätsmessung kann außer in dem zuvor beschriebenen Zusammenhang auch bei Gasabsorptionsmessungen im sichtbaren Licht oder anderen Wellenlängenbereichen angewandt werden, solange das Zielgas eine im Vergleich zu Störeinflüssen schmale Absorptionsbande aufweist, auf die ein optischer Bandpassfilter gelegt wird.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend anhand von Ausführungsbeispielen unter Bezugnahme auf die Zeichnungen beschrieben, wobei zeigen:

Fig. 1          eine schematische Darstellung eines ersten Ausführungsbeispiels einer erfindungsgemäßen Vorrichtung für den Seitenstrom,

Fig. 2 a) - c)    Prinzipdarstellungen für Strahlengänge für die Kapnometrie

Fig. 3 a), b)    schematische Darstellungen erfindungsgemäßer Vorrichtungen mit optisch neutralen Transmissionsgittern,

Fig. 4 a) - f)    schematische Darstellungen erfindungsgemäß einsetzbarer optisch neutraler Reflexionsgitter,

Fig. 5          eine schematische Darstellung eines zweiten Ausführungsbeispiels einer erfindungsgemäßen Vorrichtung,

Fig. 6          spektrale Verläufe zweier Arbeitspunkte einer Lichtquelle sowie Filter- und Absorptionsverlauf für eine Kapnometrie-Anwendung,

Fig. 7          eine schematische Darstellung eines dritten Ausführungsbeispiels einer erfindungsgemäßen Vorrichtung,

Fig. 8 a), b)    schematische Darstellungen erfindungsgemäß einsetzbarer Leistungsregelungsschaltungen,

Fig. 9          eine schematische Darstellung eines vierten Ausführungsbeispiels einer erfindungsgemäßen Vorrichtung,

Fig. 10        eine Darstellung hochaufgelöster spektraler Verläufe des Absorptionsbandes von $CO_2$ und eines erfindungsgemäß verwendbaren Bandfilters und

Fig. 11 a)-d)    eine schematische Darstellung einer MikroGlühfadenlampe.

In den Zeichnungen sind jeweils gleiche oder gleichartige Elemente und/oder Teile mit denselben Bezugsziffern versehen, so dass von einer erneuten Vorstellung jeweils abgesehen wird.

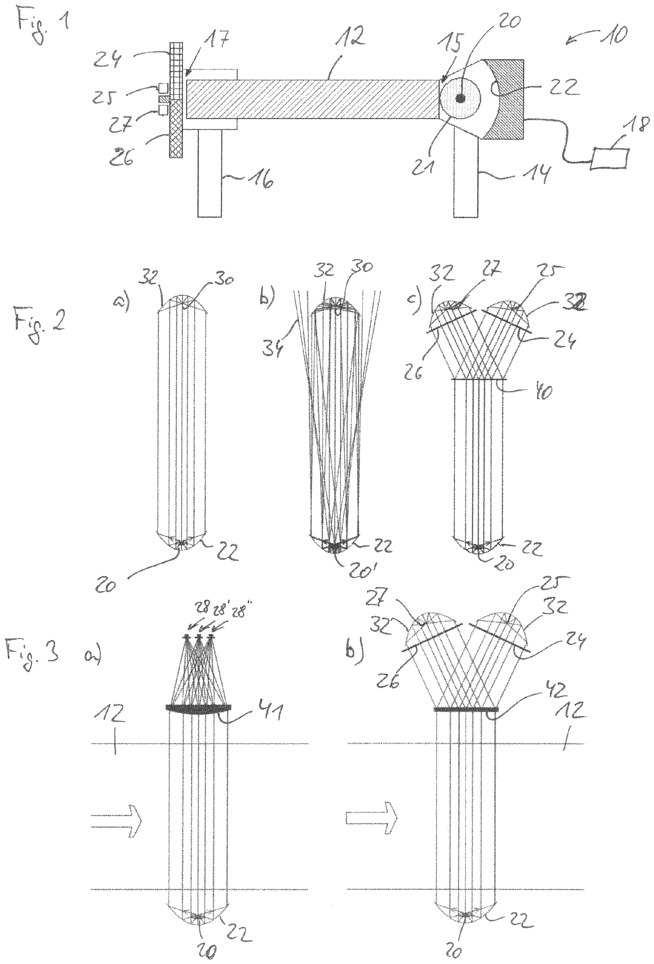

In Fig. 1 ist ein erstes Ausführungsbeispiel einer erfindungsgemäßen Anordnung 10 für den Seitenstrom schematisch dargestellt. Es kann sich dabei um ein Kapnometer handeln, welcher eine Messzelle 12 umfasst, die über einen Gaseinlass 14 und einen Gasauslass 16 mit einem konstanten Strom von Atemluft versorgt wird über eine nicht dargestellte Pumpe, welches aus einem Hauptstrom von Atemgas eines Patienten abgezweigt worden ist. Dazu verfügt die Messzelle 12 weiterhin über eine Gaseintrittsöffnung 15 und an ihrer gegenüberliegenden Seite über eine Gasaustrittsöffnung 17. Die Gasaustrittsöffnung 17 ist vorzugsweise vollständig um die Messzelle 12 herum angeordnet, so dass der erforderliche Querschnitt des Gasauslasses bei einem minimalen Abstand zwischen Messzelle und Filter realisiert werden kann. Die Messzelle 12 ist zylindrisch ausgeführt mit einem Durchmesser, der gegenüber der Länge der Messzelle 12 klein ist. Damit wird das Volumen der Messzelle 12 gegenüber der verfügbaren Absorptionslänge, also der Länge der Messzelle 12, minimiert. Die Volumenminimierung hat den Vorteil, dass das Messgas nur eine sehr kleine Verweildauer in der Messzelle 12 hat und somit eine sehr scharfe und zeitlich hochaufgelöste Messung der Konzentration eines Zielgases im Messgas ermöglicht wird.

Für die Messung umfasst die Anordnung 10 eine quasi-punktförmige Filamentquelle in Form einer Mikro-Glühfadenlampe 20, die in einem evakuierten Glaskolben angeordnet ist. Dadurch ist es möglich, die Lichtquelle bei einer sehr hohen Leistung und sehr hohen Temperatur strahlen zu lassen, ohne ihre Lebensdauer negativ zu beeinflussen. Der Hauptanteil des ausgestrahlten Lichts liegt für die Kapnometrie im Infrarotbereich, insbesondere im mittleren Infrarotbereich. Die Mikro-Glühfadenlampe 20 liegt im Brennpunkt eines kugelförmigen oder parabolischen Reflektors 22, der eine weitgehende Parallelisierung der Lichtstrahlen bewirkt, sodass die Messzelle 12 möglichst gleichmäßig durchstrahlt wird. Die Messzelle 12 kann von innen verspiegelt sein.

Ausgangs der Messzelle 12 befindet sich eine Anordnung aus zwei Detektoren 25, 27, denen zwei Filter 24, 26 vorgeschaltet sind, und die durch das Licht, das die Messzelle 12 durchstrahlt, möglichst gleichmäßig beleuchtet werden. Dem Detektor 25 ist ein Filter 24 für einen Gaskanal vorgeschaltet, welcher einen schmalen Bandpass für die Absorptionsbande des Zielgases aufweist, während der Filter 26 als Bandpassfilter für einen Referenzkanal ausgebildet ist, wo das Zielgas keine oder nur wenig Absorption hat. Nicht dargestellt ist eine Steuerung und Auswerteeinheit. Die Verwendung einer quasi-punktförmigen Lichtquelle in Form einer Mikro-Glühfadenlampe 20 in diesem Fall ermöglicht es, mit sehr wenig Lichtverlust eine sehr hohe Präzision der Messung zu erreichen, ebenfalls eine sehr schnelle und exakte Leistungs- und Temperaturregelung der Lichtquelle zu verwirklichen.

Ebenfalls ist symbolisch eine Auswertevorrichtung 18 dargestellt, die Signale von den Detektoren 25, 27 empfängt und gemäß interner Rechenregeln, look-up-Tabellen o.ä. und entsprechender Kalibrierung die Konzentration des Zielgases in der Messzelle 12 ermittelt.

In Fig. 2 a) - c) sind zur Veranschaulichung Prinzipdarstellungen für Strahlengänge für die Kapnometrie dargestellt. Figur 2 a) zeigt die Grundform für einen Kanal, bei dem eine quasi-punktförmige Infrarot-Lichtquelle, insbesondere eine Mikro-Glühfadenlampe 20, im Brennpunkt eines parabolischen Reflektors 22 angeordnet ist, der aus den vom im Brennpunkt des Reflektor 22 ausgesandten Lichtstrahlen ein paralleles Lichtstrahlenbündel macht. Dieses parallele Lichtstrahlenbündel wird vom Reflektor 32, der ebenfalls als Parabolreflektor ausgebildet ist, wiederum auf den Brennpunkt des Reflektors 32 gebündelt, in dem ein, vorzugsweise ebenfalls kleinvolumiger, Sensor 30 angeordnet ist, beispielsweise eine Photodiode. Nur sehr wenig Licht geht bei dieser Lichtübertragung verloren, sodass mit relativ geringer Ausgangsintensität einen gutes Signal-Rausch-Verhältnis (SNR) erzielt werden kann.

Im Gegensatz dazu ist in Figur 2 b) der Fall dargestellt, dass die Lichtquelle 20' nicht quasi-punktförmig ist. In diesem Fall liegt ein Teil der Lichtquelle 20' außerhalb des Brennpunkts des Reflektors 22, sodass das ausgesandte Licht nicht vollständig als paralleles Lichtbündel zum gegenüberliegenden Reflektor 32 übermittelt wird, sondern in nicht paralleler Weise am Reflektor 32 vorbeiläuft und somit verloren geht. Dieser Lichtverlust sorgt dafür, dass die Menge und Intensität an ausgestrahltem Licht größer sein muss als im erfindungsgemäßen Fall, was zu einer Ineffizienz und einem höheren Stromverbrauch führt.

Figur 2 c) zeigt ein weiteres Beispiel, bei dem die Lichterzeugung neben der gleichen Weise erfolgt wie in Figur 2 a) dargestellt, jedoch trifft das parallele Lichtbündel auf ein schematisch dargestelltes spektral neutrales Transmissionsgitter 40, welches das Lichtbündel ohne dispersive Effekte, also spektral neutral, auf zwei verschiedene Lichtbündel aufteilt, welche durch die entsprechenden Filter 24, 26 für einen Gaskanal und einen Referenzkanal auf zwei entsprechende Reflektoren 32 und die entsprechenden Sensoren 25, 27 für einen Gaskanal und einen Referenzkanal aufgeteilt wird.

Die Figuren 3 a), b) zeigen schematische Darstellungen der optischen Verhältnisse erfindungsgemäßer Vorrichtungen mit spektral neutralen Transmissionsgittern. In Figur 3 a) ist ein Fall gezeigt, wo die Lichterzeugung so erfolgt wie in den Figuren 2 a) und 2 c). Nach Durchqueren der Messzelle 12 erreicht das parallele Lichtbündel ein spektral neutrales Transmissionsgitter 41, dass zusätzlich durch eine entsprechende Wölbung auch noch fokussierend wirkt. In der gezeigten Ausführungsform wird das parallele Lichtbündel durch das Fokussieren des spektral neutralen Transmissionsgitters 41 auf drei verschiedene kleinflächige Sensoren 28, 28', 28", die beispielsweise für einen Referenzkanal und zwei verschiedene Zielgase mit unterschiedlichen Absorptionsbereichen ausgelegt sein können, alternativ auch für drei verschiedene Zielgase oder auch für ein Gas und zwei Referenzwellenlängen, was dann eine Dispersionskompensation ermöglicht.

Im dargestellten Fall durchquert das Licht die Messzelle 12 quer zur Strömungsrichtung des Gasgemisches. Dies ist sowohl im Hauptstrom als auch im Nebenstrom anwendbar. Für eine Verwendung im Seitenstrom kann aber auch die Ein- und Auskopplung in die Messzelle 12 bzw. aus dieser heraus kollinear mit der Hauptströmungsrichtung in der Messzelle 12 eingestellt werden.

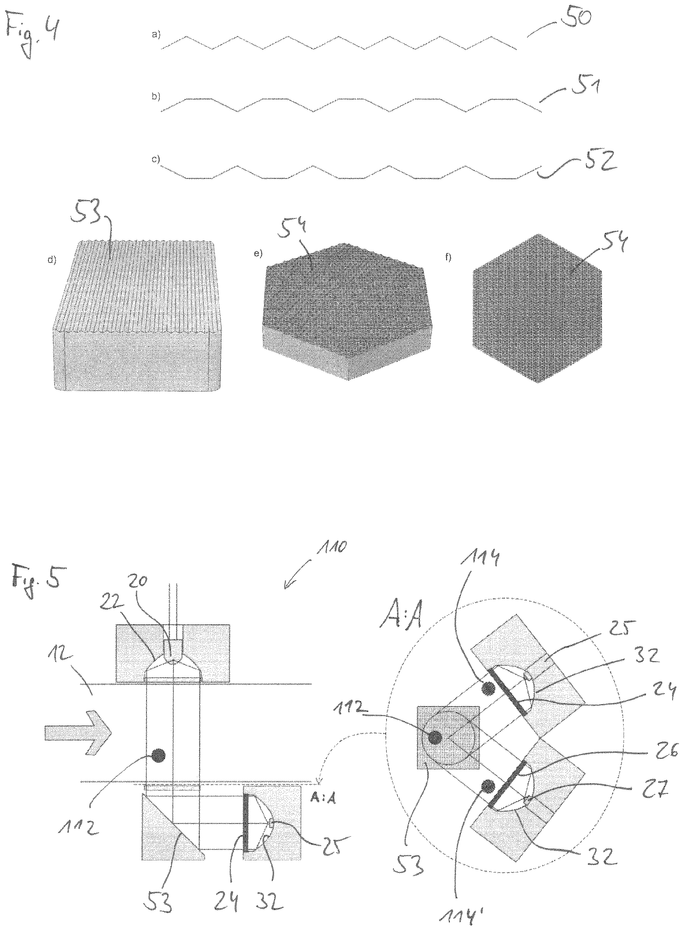

In Fig. 4 a) - f) sind schematische Darstellungen erfindungsgemäß einsetzbarer spektral neutraler Reflexionsgitter gezeigt. Die im Querschnitt gezeigte Struktur 50 aus Figur 4 a) dient der Aufteilung des Lichts auf zwei Detektoren, die Strukturen 51, 52 aus Figur 4 b) und 4 c) der Aufspaltung des Lichts auf drei Detektoren. In Figur 4 d) ist eine dreidimensionale spezifische Darstellung eines Reflexionsgitters 53 gezeigt, die mit ihrer Satteldachstruktur, ähnlich wie Struktur 50 aus Figur 4 a) der Aufteilung auf zwei Detektoren dient. In Figur 4 e) ist in einer perspektivischen Darstellung und in Figur 4 f) in einer frontalen Draufsicht eine Oberflächenstruktur eines Reflexionsgitters 54 gezeigt, welches auf einer Pyramidenstruktur mit hexagonalem Grundgerüst beruht. Dieses Reflexionsgitter 54 ist für eine Aufteilung des Lichts auf sechs Detektoren geeignet.

Die Fig. 5 zeigt eine schematische Darstellung eines zweiten Ausführungsbeispiels einer erfindungsgemäßen Anordnung 110, und zwar im linken Teilbild in einer Seitenansicht und im rechten Teil in einer frontalen Draufsicht gemäß Ebene A:A aus dem linken Teilbild. Die Mikro-Glühfadenlampe 20 ist in einem Halter eingefasst, der einen parabolischen Reflektor 22 aufweist, der das ausgesandte Licht in ein paralleles Strahlenbündel umwandelt und durch die Messzelle 12 der Anordnung 110 strahlt, welches von links (in Pfeilrichtung) mit Messgas durchströmt wird. Diese Anordnung entspricht daher eher einer Messung im Hauptstrom, jedoch ist sie auch in geeigneter Weise für eine Seitenstrommessung verwendbar, oder durch entsprechende Ein- und Auskopplung des Lichts in die Messzelle 12 in der Hauptströmungsrichtung für eine Seitenstrommessung optimierbar.

Nach Austritt aus der Messzelle 12 wird das Lichtbündel über ein Reflexionsgitter 53 entsprechend Figur 4 b) in der gezeigten Ansicht um 90° abgelenkt, wobei in diesem Fall als Detektionseinheit eine Gruppierung aus einem Filter 24 für den Gaskanal und ein Detektor 25 für den Gaskanal des Zielgases dargestellt ist, wobei der kleinflächige Detektor 25 im Brennpunkt des parabolischen Reflektors 32 angeordnet ist. Zum Schutz der einzelnen Bauteile sind außerdem noch drei nicht bezeichnete, optisch durchlässige und spektral neutrale Eintritts- und Austrittsfenster dargestellt.

In der rechten Darstellung in Figur 5 ist erkennbar, wie das Reflexionsgitter 53 das als Kreis gezeichnete Lichtbündel auf zwei verschiedene Lichtbündel für zwei gleichartig aufgebaute Detektoreinheiten für den Absorptionskanal und den Referenzkanal aufteilt. Die Bezugszeichen entsprechen denjenigen aus Figur 2 c) und Figur 3 b).

Ein Vorteil dieses Aufbaus für den Fall von Verschmutzungen im optischen Pfad ist in Figur 5 ebenfalls gezeigt. In der Messzelle hat sich eine Störung 112 gebildet, beispielsweise ein Kondensattropfen oder ein Schmutzpartikel, der stationär oder mobil in der Messzelle 12 angeordnet ist. Damit eine solche Störung die Messung nicht beeinträchtigt, sorgt das Reflexionsgitter 53 dafür, dass diese Störung gleichartig in beiden Kanälen, also dem Absorptionskanal und dem Referenzkanal, vorhanden ist. Dies ist in der rechten Darstellung in Figur 5 zu sehen, wo die Abbilder 114, 114' der Störung 112 in den beiden Kanälen in gleicher Art und Weise auftauchen und in gleicher Art und Weise zu einer Abschwächung des Signals führen. Da die Konzentrationsmessung über die Absorption, also den Vergleich der Intensitäten im Absorptionskanal und im Referenzkanal miteinander erfolgt, sind beide Kanäle in gleicher Weise betroffen. Die Strukturierung der Abbilder 114, 114' ist ein Resultat der Aufteilung des gesamten Lichts auf die beiden Detektoren 25, 27 durch die Satteldachstruktur des Reflexionsgitters 53. Da beide Kanäle betroffen sind, kürzt sich dieser Beitrag im Wesentlichen heraus, ohne das Verhältnis der gemessenen Intensitäten wesentlich zu beeinträchtigen.

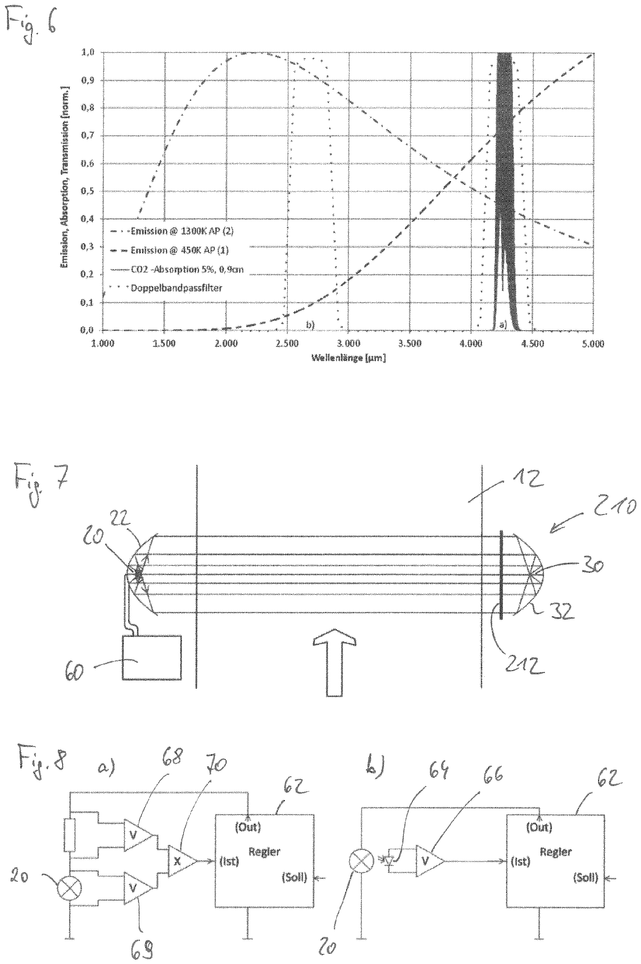

In Fig. 6 sind die spektralen Verläufe zweier Arbeitspunkte einer Lichtquelle sowie Filter- und Absorptionsverlauf für eine Kapnometrie-Anwendung dargestellt. Auf der horizontalen Achse ist die Wellenlänge im Bereich von 1 bis 5 $\mu$m dargestellt, also der Infrarotbereich, auf der vertikalen Achse normalisierte Werte für die Emission, Absorption und Transmission, zwischen 0 und 1. Gemäß der Legende unten links ist die $CO_2$-Absorption einer 5-prozentigen Konzentration und einer Absorptionslänge von 0,9 cm bei ca. 4,26 $\mu$m als durchgezogene Linie dargestellt. Um diese Absorptionsbande herum ist ein Bereich a) eines Doppelbandpassfilters gezogen, der Infrarotlicht im Bereich von ca. 4,1 bis 4,4 $\mu$m durchlässt, so dass der gesamte Bereich, der in $CO_2$ absorbiert wird, in diesen Bereich a) des Doppelbandpassfilters fällt. Ein zweiter Bereich b) des Doppelbandpassfilters dient als Referenzfilter, der Infrarotlicht ca. zwischen 2,5 und 2,8 $\mu$m durchlässt. Hier hat $CO_2$ keine Absorption.

Mit strichpunktierter Linie und mit gestrichelter Linie ist die Emission eines thermischen Strahlers bei 1300 K bzw. 450 K in einem ersten und einem zweiten Arbeitspunkt (AP) dargestellt. Bei der höheren Temperatur liegt das Maximum der Lichtintensität bei ca. 2,25 $\mu$m, während der dargestellte Verlauf bei einer Temperatur des thermischen Strahlers von 450 K sein Maximum noch nicht erreicht hat. Es ist darauf hinzuweisen, dass bei höherer Temperatur auch die abgestrahlte Leistung größer ist, so dass die beiden Emissionsspektren in der Darstellung der Figur 6 sehr unterschiedliche Normierungsfaktoren zueinander haben.

Deutlich ist zu erkennen, dass bei der niedrigen Temperatur im Arbeitspunkt 1 sehr wenig Lichtintensität im Referenzband b), jedoch wesentlich mehr Intensität im Absorptionsband a) vorhanden ist. Im höheren Arbeitspunkt 2 bei 1300 K ist die Lichtintensität im Referenzband b) jedoch höher als im Absorptionsband a), sodass durch eine entsprechende Temperaturmodulation zwischen den Anteilen der Referenz und der Gasabsorption bei Kenntnis des jeweiligen Emissionsspektrums gut unterschieden werden kann. Dies kann im Rahmen einer Kalibration sehr gut eingestellt werden.

Fig. 7 zeigt eine schematische Darstellung eines dritten Ausführungsbeispiels einer erfindungsgemäßen Anordnung 210 in einer Doppelbandpassanordnung, so dass die Spektrumsverhältnisse aus Figur 6 in diesem Fall zum Tragen kommen. Die optische Anordnung des einen Kanals, mit der die Messzelle 12 durchstrahlt wird, entspricht derjenigen aus Figur 2 a). Auch diese Anordnung ist im Hauptstrom oder im Nebenstrom verwendbar, und durch geeignete Ein- und Auskopplung des Lichts in die Messzelle 12 kann das Licht in der Strömungsrichtung der Messzelle geführt werden.

In dem in Fig. 7 gezeigten Fall wird das von der Mikro-Glühfadenlampe 20 ausgesendete Licht nach Durchquerung der Messzelle 12 durch einen Doppelbandpassfilter 212 mit der Spektralcharakteristik aus Figur 6 gefiltert und auf den einzelnen Sensor 30 gebündelt. Dazu ist eine Steuervorrichtung 60 mit einer Leistungsregelung vorgesehen, die die Leistungsabgabe und Temperatur des quasi-punktförmigen thermischen Strahlers, also der Mikro-Glühfadenlampe 20, sehr genau steuert, um die entsprechenden Emissionsspektren aus Figur 6 oder geeignet gewählte andere Temperaturen mit den entsprechenden Emissionsspektren sehr schnell modulierend einzustellen.

Fig. 8 a), b) stellt hierzu erfindungsgemäß einsetzbare Leistungsregelungsschaltungen schematisch dar. In Figur 8 a) ist eine Multiplikationsschaltung verwirklicht, bei der mit 2 Verstecken 68, 69 ein momentaner Strom und ein momentaner Spannungsabfall über der Mikro-Glühfadenlampe 20 gemessen werden, Strom und Spannung im Multiplikator 70 miteinander multipliziert werden und als Istwert in eine Regeleinheit 62 eingegeben werden, die mit einem Sollwert verglichen wird und als Ausgangswert (Out) wiederum die Lichtquelle über einen Vorwiderstand ansteuert.

Diese Messung von Strom und Widerstand sowie die Multiplikation kann entweder analog oder digital erfolgen.

Alternativ kann gemäß Figur 8 b) die von der Lichtquelle 20 abgegebene Lichtleistung auch über eine Photodiode 64 gemessen werden, deren Ausgangsstrom wiederum über einen Verstärker 66 als Istwert in die Regeleinheit 62 eingespeist wird, die entsprechend die Leistung der Mikro-Glühfadenlampe 20 dann auf den variablen Sollwert regelt.

Die Lösung gemäß Figur 8 b) ist einfacher als diejenige aus Figur 8 a), jedoch ist zu beachten, dass ein Ausschnitt des Spektrums der Lichtquelle für die Regelung der Lichtquelle genutzt wird, der nicht der $CO_2$-Messung dient, jedoch zu ihr proportional ist. Zusätzlich ist es günstig, für die Regelung eine, gegenüber der Messwellenlänge kurzwellige Photodiode, beispielsweise im visuellen Spektrum oder im nahen Infrarot, zu nutzen, da diese eine deutlich stabilere Regelung ermöglichen. Diese Photodioden weisen üblicherweise ein im Vergleich zum Emissionsspektrum der Mikro-Glühfadenlampe 20 schmalerbandiges Spektrum auf, so dass die Sollwerte entsprechend angepasst werden müssen. Gegebenenfalls sind auch Nichtlinearitäten zu berücksichtigen, die durch die Überlagerung des jeweiligen temperaturabhängigen Emissionsspektrums mit dem Empfindlichkeitsspektrum des Sensors entstehen.

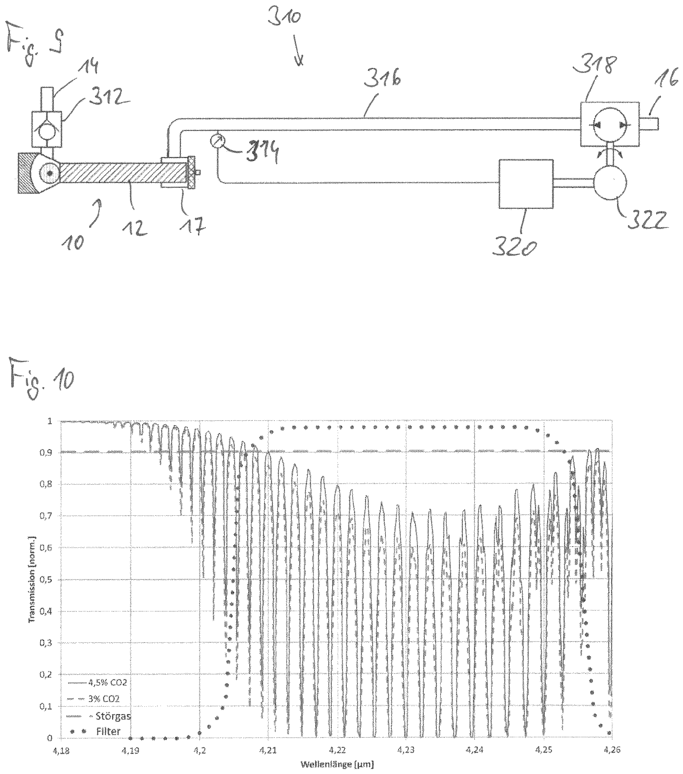

Fig. 9 zeigt eine schematische Darstellung eines vierten Ausführungsbeispiels einer erfindungsgemäßen Anordnung 310, welches mit jedem der vorherigen Ausführungsbeispiele gekoppelt werden kann, und zwar sowohl mit solchen Ausführungsbeispielen, die nur einen optischen Kanal aufweisen, als auch mit solchen Ausführungsbeispielen, in denen das Lichtbündel nach Durchquerung der Messzelle 12 auf zwei getrennte optische Kanäle aufgeteilt wird. Rein beispielhaft ist als Grundlage der Anordnung 310 eine Anordnung 10 gemäß dem ersten Ausführungsbeispiel aus Figur 1 gewählt worden. Eine Modifikation besteht darin, dass der Gaseinlass 14 mit einem Rückschlagventil 312 ausgestattet ist, welches in Gaseinlassrichtung öffnet und entgegen dieser Richtung sperrt. Ausgangs der Messzelle 12 und der Gasaustrittsöffnung 17 schließt sich ein zu einem Gasreservoir 316 erweiterter Gasauslass 16 an, der mit einem Druckmesser 314 zur Messung des Gasdrucks in dem Gasreservoir 316 ausgestattet ist, wobei der gemessene Druck auch dem Druck in der Messzelle 12 entspricht.

Ferner ist eine Pumpe 318 vorgesehen, welche Gase entweder in das schlanke Gasreservoir 316 und die Messzelle 12 hineinpumpen kann oder in umgekehrter Pumprichtung betrieben werden kann, um Gas aus der Messzelle 12 herauszuleiten und somit den Druck in einer Pumprichtung zu erhöhen und in der entgegengesetzten Pumprichtung abzusenken. Hierfür ist ein Regler 320 für die Pumpe 318 vorgesehen, der über einen Motor 322, der als Stellmotor ausgebildet sein kann oder direkt den Pumpenrotor regelt, und die Pumprichtung und/oder die Pumpstärke der Pumpe 318 beeinflusst. Mit dieser Anordnung 310 ist es möglich, eine Nichtlinearitätsanalyse in Abhängigkeit des in der Messzelle 12 vorhandenen Drucks durchzuführen, wie sie zuvor beschrieben wurde, beispielsweise gemäß den Formeln (8) und (9). So ist es u.a. möglich, zyklisch wiederkehrend eine Druckveränderung in der Messzelle 12 zu erreichen, die als unabhängige Analyse dazu verwendet werden kann, um zu überprüfen, ob die Kalibrationsparameter der zugrunde liegenden kontinuierlichen Messung noch stimmen oder angepasst werden müssen, da mithilfe dieser Methode die Konzentration des Zielgases isoliert von Störquellen bestimmt werden kann. Da diese Nichtlinearitätsmessung die beste Genauigkeit bei hohen Konzentrationen des Zielgases aufweist, ist es bevorzugt, die Druckmodulation dann vorzunehmen, wenn, beispielsweise beim Ausatmen, der endexpiratorische Wert, typischerweise ca. 5 % $CO_2$, erreicht ist.

Die für diese Analyse zugrunde liegenden Spektralverläufe sind in Fig. 10 in hoher Auflösung dargestellt. Der auf der horizontalen Achse dargestellte Wellenlängenbereich zwischen 4,18 und 4,26 $\mu$m entspricht einem schmalen Ausschnitt aus dem in Figur 6 dargestellten Spektrumsbereich. Deutlich zu sehen ist die Bandenstruktur der $CO_2$-Absorption, in diesem Fall in einer Transmissionsdarstellung. Im Gegensatz zu dem Doppelbandpassfilter aus Figur 6 kommt in diesem Fall ein schmalerer Bandpassfilter zum Einsatz, der Randbereiche der Absorption von $CO_2$ abschneidet und somit vergleichsweise etwas schmaler ist als das $CO_2$-Absorptionsband selbst. Eine zu kleine Bandbreite würde die verfügbare Lichtintensität in unerwünschter Weise verringern, jedoch ist mit der gezeigten Konfiguration eine recht starke Nichtlinearität in der Druckabhängigkeit gewährleistet. Die Intensität des beim Detektor Ankommenden resultiert mathematisch betrachtet aus einer Überlagerung der Filterfunktion mit der wellenlängenabhängigen Funktion des Absorptionskoeffizienten des Zielgases mit seiner Bandenstruktur. Diese Überlagerung führt zu einer nichtlinearen Abhängigkeit der detektierbaren Transmission von dem Druck bzw. der Konzentration des Zielgases.

Ebenfalls eingezeichnet ist ein als im dargestellten Bereich im Wesentlichen konstant angenommener Absorptionskoeffizient eines Störgases, welcher die durchgestrahlte Lichtleistung ebenfalls vermindert. Da allerdings der Absorptionskoeffizient des Störgases wellenlängenunabhängig ist, ergibt die Überlagerung der Filterfunktion der Störgastransmission in diesem Bereich lediglich lineare, jedoch keine nichtlinearen druckabhängigen Terme.

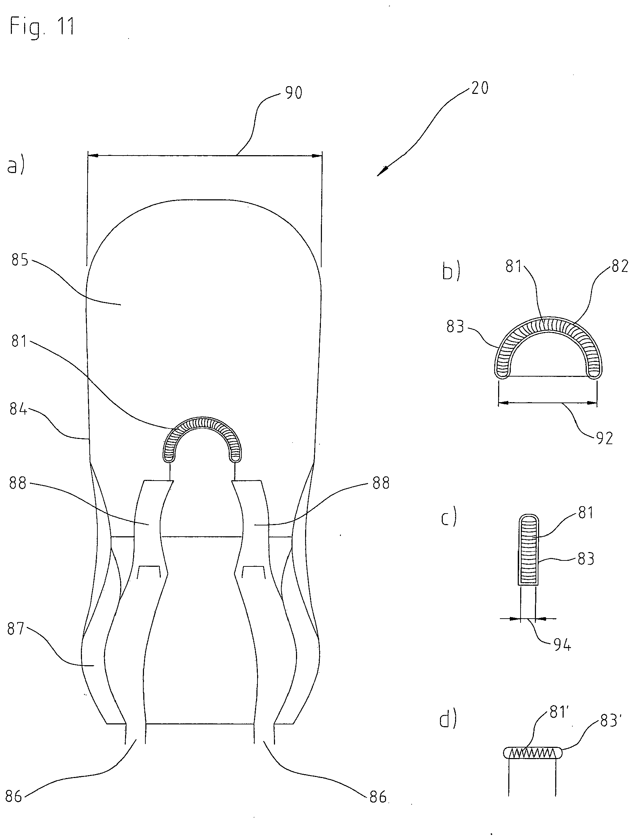

In Fig. 11 a) ist eine erfindungsgemäß einsetzbare Mikro-Glühfadenlampe 20 beispielhaft schematisch dargestellt. Diese umfasst als zentrale Lichterzeugungseinheit eine Wendel 81, die in einer evakuierten oder mit einem Schutzgas gefüllten Kapsel 84 angeordnet ist. Der Bereich um die Wendel 81 herum ist mit einem Vakuum 85 ausgestattet. Die Kapsel 84 selbst ist durchsichtig, beispielsweise aus Glas. Der evakuierte Innenraum wird nach unten hin abgeschlossen durch einen massiven Sockel 87, der von zwei Zuleitungen 86 durchdrungen wird, die in Kontaktstifte 88 münden, an denen die Wendel 81 jeweils kontaktiert ist. Die Kapsel 84 hat eine im wesentlichen zylindrische Gestalt, deren Durchmesser 90 in Bezug auf die Längsachse der Mikro-Glühfadenlampe 20 genommen wird. Diese beträgt vorteilhafterweise weniger als 2 mm, wobei noch kleinere Kapseln 84 zur optimalen Leistungsausnutzung noch vorteilhafter sind.

Die Wendel 81 hat eine gebogene Form, sodass auf relativ geringem Raum viel Wendellänge zur Verfügung steht und somit eine hohe Leistungsdichte erreicht wird. In Figur 11 b) ist die Wendel 81 zusammen mit ihrer Einhüllenden 83 gezeigt, wobei in der schematischen Darstellung insofern nur zur Verdeutlichung ein Abstand zwischen der Einhüllenden 83 und der Wendel 81 gelassen ist. Die Einhüllende umgrenzt das Volumen 82, das von der Wendel 81 aufgespannt wird. Die größte lineare Erstreckung der Einhüllenden 92 verläuft in diesem Fall zwischen den beiden Endpunkten der Wendel 81 und somit zum großen Teil außerhalb der Einhüllenden 83 der Wendel 81. Diese größte lineare Erstreckung 92 beschreibt aber auch den größten bzw. größtmöglichen linearen Abstand zwischen zwei Punkten der Wendel 81. Insofern ist diese größte lineare Erstreckung 92 ein lineares Maß für die Kompaktheit des lichterzeugenden Bereichs der Mikro-Glühfadenlampe 20. Speziell in einer abbildenden Optik ist eine möglichst kleine größte lineare Erstreckung 92 vorteilhaft, weil ein Großteil des erzeugten Lichts im Brennpunkt der abbildenden Optik angeordnet werden kann, sodass Lichtverluste vermieden werden können. Dies geht zugunsten des Signal-Rausch-Abstands und der Akkulaufzeit.

Gleichzeitig weist eine entsprechende Wendel 81 eine sehr geringe thermische Masse auf, so dass das Aufheizen der Wendel auf Betriebstemperatur in Sekundenbruchteilen erfolgt und auch eine Modulation um mehrere 100 °C mit einer ausreichenden Frequenz für eine zeitauflösende Messung der Änderung der Gaskonzentration eines Messgases ausführbar ist, beispielsweise für die Verfolgung der Konzentration von $CO_2$ in einem Atemgas im Rahmen einer Kapnometrie.

In Figur 11 c) ist die Wendel 81 von der Schmalseite her gezeigt. In diesem Fall ist die Dicke der Wendel gleich der kleinsten linearen Erstreckung 94 der Einhüllenden 83. In Figur 11 d) ist eine Alternative Wendel 81' gezeigt, die, anders als die Wendel 81, nicht gebogen, sondern gerade ist. Die Einhüllende 83' ist in diesem Fall im Wesentlichen zylindrisch, und die größte lineare Erstreckung der Einhüllenden liegt in der Wendel 81'. Bei gleicher absoluter Länge der Wendeln ist die lineare Ausdehnung der Wendel 81' daher größer als in dem zuvor gezeigten Fall der Wendel 81. Eine Biegung der Wendel ist somit im Rahmen der vorliegenden Erfindung vorteilhaft, um eine hohe Lichtintensität aus einem kleinen Gesamtvolumen zu erzeugen.

Die zuvor gezeigten Ausführungsbeispiele zeigen jeweils den Idealfall einer Anordnung beispielsweise mit Aufteilung durch ein Transmissionsgitter oder Reflexionsgitter, welches jeweils spektral neutral ist, eine Doppelbandpassfilter-Anordnung und eine Anordnung für die Nichtlinearitätsanalyse. Diese Anordnungen lassen sich allerdings auch miteinander kombinieren, sodass auch die Nichtlinearitätsanalyse beispielsweise in einer Doppelbandpassfilter-Anordnung oder einer Anordnung mit spektral neutralem Gitter und mehreren Empfängern anwendbar ist. Ebenfalls lässt sich eine Doppelbandpassfilter-Anordnung mit spektral neutralen Transmissionsgittern oder Reflexionsgittern und mehreren Detektoren kombinieren, um beispielsweise eine Analyse in Bezug auf mehrere Zielgase durchzuführen.

Der Schutzumfang der Erfindung wird durch die angehängten Ansprüche definiert.

Bezugszeichenliste

| | |
|---|---|
| 10 | Anordnung |
| 12 | Messzelle |
| 14 | Gaseinlass |
| 15 | Gaseintrittsöffnung |
| 16 | Gasauslass |
| 17 | Gasaustrittsöffnung |
| 18 | Auswertevorrichtung |
| 20 | Mikro-Glühfadenlampe |
| 20' | ausgedehnte Lichtquelle |
| 21 | evakuierter Glaskolben |
| 22 | Reflektor |
| 24 | Filter für Gaskanal |
| 25 | Detektor für Gaskanal |
| 26 | Filter für Referenzkanal |
| 27 | Detektor für Referenzkanal |
| 28, 28', 28" | Detektoren |
| 30 | IR-Photodiode |
| 32 | Reflektor |
| 34 | verlorene Lichtanteile |
| 40 | spektral neutrales Transmissionsgitter |
| 41 | spektral neutrales fokussierendes Transmissionsgitter |

| 42 | spektral neutrales Transmissionsgitter |
| 50 - 54 | spektral neutrales Reflexionsgitter |
| 60 | Steuervorrichtung |
| 62 | Regeleinheit |
| 64 | Photodiode |
| 66 | Verstärker |
| 68 | Verstärker für Strommessung |
| 69 | Verstärker für Spannungsmessung |
| 70 | Multiplikator |
| 81 | Wendel |
| 82 | Volumen |
| 83, 83' | Einhüllende |
| 84 | Kapsel |
| 85 | Vakuum |
| 86 | Zuleitung |
| 87 | Sockel |
| 88 | Kontaktstift |
| 90 | Durchmesser der Kapselung |
| 92 | größte lineare Erstreckung der Einhüllenden |
| 94 | kleinste lineare Erstreckung der Einhüllenden |
| 110 | Anordnung |
| 112 | Störung |
| 114, 114' | Abbild der Störung |
| 210 | Anordnung |
| 212 | Doppelbandpassfilter |
| 310 | Anordnung |
| 312 | Rückschlagventil |
| 314 | Druckmesser |
| 316 | Gasreservoir |
| 318 | Pumpe |
| 320 | Regler für die Pumpe |
| 322 | Motor |

## Patentansprüche

1. Anordnung (10, 110, 210, 310) zum Messen einer Gaskonzentration mittels Absorptionsspektroskopie, insbesondere zur kapnometrischen Messung des Anteils von $CO_2$ in Atemluft, welche so ausgebildet ist, dass im Betrieb IR-Licht von einer thermischen Lichtquelle durch eine Messzelle (12) mit einem zu analysierenden Gasgemisch geleitet wird, und ferner ausgebildet ist, die Gaskonzentrationen eines in dem Gasgemisch enthaltenen zu messenden Gases über eine Messung einer Abschwächung des in die Messzelle (12) eingeleiteten Lichts aufgrund von Absorption in dem zu messenden Gas zu bestimmen, wobei die Anordnung (10, 110, 210, 310) einen optischen Strahlengang mit einer IR-Licht erzeugenden thermischen Lichtquelle und einem zugeordneten kugelförmigen oder parabolischen Reflektor (22), eine Messzelle (12), welche mit dem Gasgemisch füllbar oder gefüllt ist und eine Messstrecke für das von der thermischen Lichtquelle erzeugte Licht, wenigstens einen Sensor (25, 27, 28, 28', 28", 30) sowie wenigstens einen Bandpassfilter (24, 26, 212), der dem wenigstens einen Sensor (25, 27, 28, 28', 28", 30) vorgeschaltet ist, aufweist, wobei die Anordnung (10, 110, 210, 310) ferner eine Auswertevorrichtung (18) umfasst, die zur Bestimmung der zu messenden Gaskonzentration aus der Abschwächung des IR-Lichts in der Messzelle (12) ausgebildet ist, wobei

- wenigstens zwei Sensoren umfasst sind und der wenigstens eine Bandpassfilter (24, 212) zur Transmission in einem Messwellenlängenbereich ausgebildet ist, in dem das zu messende Gas IR-Licht absorbiert, und der wenigstens eine Bandpassfilter (212) oder wenigstens ein weiterer Bandpassfilter (26) zur Transmission in einem Referenzwellenlängenbereich ausgebildet ist, in dem das zu messende Gas IR-Licht nicht oder im Vergleich zum Messwellenlängenbereich nur wenig absorbiert, oder
- nur ein einzelner Sensor (30) umfasst ist und der dem einzelnen Sensor (30) vorgeschaltete Bandpassfilter als Doppelbandpassfilter (212) ausgebildet ist, der IR-Licht sowohl im Messwellenlängenbereich als auch im Referenzwellenlängenbereich durchlässt,

wobei die thermische Lichtquelle als Mikro-Glühfadenlampe (20) mit einer in einer evakuierten oder mit einem Schutzgas gefüllten Kapsel (84) angeordneten lichterzeugenden Wendel (81) ausgebildet und im Brennpunkt des kugelförmigen oder parabolischen Reflektors (22) angeordnet ist, wobei der wenigstens eine Sensor (25, 27, 28, 28', 28", 30) als infrarotempfindliche Photodiode ausgebildet ist, deren sensitive Fläche weniger als 1 mm$^2$ beträgt.

2. Anordnung (10, 110, 210, 310) nach Anspruch 1, **dadurch gekennzeichnet, dass** die Kapsel (84) der Mikro-Glühfadenlampe (20) einen Durchmesser (90) von weniger als 2 mm, insbesondere weniger als 1,5 mm, insbesondere weniger als 1 mm, aufweist.

3. Anordnung (10, 110, 210, 310) nach Anspruch 1 oder 2, **dadurch gekennzeichnet, dass** ein größter linearer Abstand zwischen zwei Punkten der Wendel (81) weniger als 1 mm, insbesondere weniger als 0,5 mm, beträgt.

4. Anordnung (10, 110, 210, 310) nach einem der Ansprüche 1 bis 3, **dadurch gekennzeichnet, dass** eine Einhüllende der Wendel (81) in einer Projektionsrichtung, in der die Einhüllende eine maximale Einhüllenden-Projektionsfläche annimmt, eine maximale Einhüllenden-Projektionsfläche von weniger als 0,1 mm$^2$, insbesondere weniger als 0,02 mm$^2$, aufweist.

5. Anordnung (10, 110, 210, 310) nach einem der Ansprüche 1 bis 4, **dadurch gekennzeichnet, dass** die sensitive Fläche des Sensors (25, 27, 28, 28', 28", 30) oder der Sensoren (25, 27, 28, 28', 28", 30) kleiner als 0,15 mm$^2$ ist.

6. Anordnung (10, 110, 210, 310) nach einem der Ansprüche 1 bis 5, **dadurch gekennzeichnet, dass** eine Steuerungsvorrichtung (60) umfasst ist, die zur leistungsgeregelten Ansteuerung der Mikro-Glühfadenlampe (20) ausgebildet ist, wobei die Steuerungsvorrichtung (60), um einen Ist-Wert der abgegebenen Leistung zu bestimmen, zur Bildung eines Produktes aus an der Mikro-Glühfadenlampe (20) gemessenem Strom und gemessener Spannung ausgebildet ist oder mit einer bei der Mikro-Glühfadenlampe (20) angeordneten Photodiode (64) signalverbunden ist, die einen Teil des von der Mikro-Glühfadenlampe (20) erzeugten Lichts empfängt.

7. Anordnung (10, 110, 210, 310) nach einem der Ansprüche 1 bis 6, **dadurch gekennzeichnet, dass** das IR-Licht gebündelt durch die Messzelle (12) geleitet wird und nach Durchgang durch die Messzelle mittels eines spektral neutralen optischen planparallelen oder gewölbten Transmissions- oder Reflexionsgitters (40, 41, 42, 50 - 54) auf zwei oder mehr Sensoren (25, 27, 28, 28', 28", 30) aufgeteilt wird, wobei das Transmissionsgitter (40, 41, 42) oder Reflexionsgitter (50 - 54) eine Gitterkonstante aufweist, die um einen Faktor von 30 oder mehr, insbesondere um einen Faktor von 50 und mehr, kleiner ist als ein Durchmesser eines Lichtflecks auf dem Transmissions- oder Reflexionsgitter (40, 41, 42, 50 - 54).

8. Anordnung (10, 110, 210, 310) nach einem der Ansprüche 1 bis 7, **dadurch gekennzeichnet, dass** die Messzelle (12) als innen diffus oder hochglänzend verspiegelte Röhre ausgebildet ist, an deren einem Ende die Mikro-Glühfadenlampe (20) und an deren anderem Ende der Sensor (25, 27, 28, 28', 28", 30) oder die Sensoren (25, 27, 28, 28', 28", 30) mit den vorgeschalteten Bandpassfiltern (24, 26, 212) angeordnet sind.

9. Anordnung (10, 110, 210, 310) nach einem der Ansprüche 1 bis 8, **dadurch gekennzeichnet, dass**, wenn nur ein einzelner Sensor umfasst und der dem einzelnen Sensor vorgeschaltete Bandpassfilter als Doppel-Bandpassfilter (212) ausgebildet ist, der IR-Licht sowohl im Messwellenlängenbereich als auch im Referenzwellenlängenbereich durchlässt, die Steuerungsvorrichtung (60) ausgebildet und eingerichtet ist, die Mikro-Glühfadenlampe (20) zwischen einem Arbeitspunkt niedriger Leistung und einem Arbeitspunkt hoher Leistung zu modulieren, in denen das jeweilige Emissionsspektrum unterschiedliche Anteilsverhältnisse im Messwellenlängenbereich und im Referenzwellenlängenbereich aufweist.

10. Anordnung (10, 110, 210, 310) nach einem der Ansprüche 1 bis 9, **dadurch gekennzeichnet, dass** Mittel umfasst sind, die zu einer temporären Druckerhöhung und Druckabsenkung des Gasgemischs in der Messzelle (12) ausgebildet sind, insbesondere eine Pumpe (318) und/oder ein oder mehrere, insbesondere schaltbare, Ventile.

11. Verfahren zum Messen einer Gaskonzentration mittels Absorptionsspektroskopie, insbesondere zur kapnometrischen Messung des Anteils von $CO_2$ in Atemluft, mit einer Anordnung (10, 110, 210, 310) nach einem der Ansprüche 1 bis 10, in welchem IR-Licht von der Mikrofaden-Glühfadenlampe (20) als thermische Lichtquelle durch die Messzelle (12) mit einem zu analysierenden Gasgemisch geleitet wird, wenigstens eine Messung einer Abschwächung des in die Messzelle (12) eingeleiteten Lichts aufgrund von Absorption in einem in dem Gasgemisch enthaltenen zu messenden Gas und wenigstens eine Messung einer spektralen Referenz durch den wenigstens einen Sensor (25, 27, 28, 28', 28", 30) durchgeführt wird und daraus die Gaskonzentration des zu messenden Gases mit der Auswertevorrichtung (18) bestimmt wird.

12. Verfahren nach Anspruch 11, **dadurch gekennzeichnet, dass** die Mikro-Glühfadenlampe (20) mit einer Messwiederholfrequenz $f_{Mess}$, die größer ist als 10 Hz, insbesondere größer als 25 Hz, moduliert wird, wobei eine Temperatur der Wendel (81) während der Messung höher ist als 400 °C und einen Temperaturmodulationshub von wenigstens 300 °C, insbesondere wenigstens 500 °C aufweist, insbesondere im Maximum 1000 °C übersteigt.

13. Verfahren nach Anspruch 11 oder 12, **dadurch gekennzeichnet, dass** die Mikro-Glühfadenlampe (20) leistungsgeregelt betrieben wird.

14. Verfahren nach einem der Ansprüche 11 bis 13, **dadurch gekennzeichnet, dass** im Laufe einer Messung in zeitlichen Abständen hintereinander der Druck des Gasgemischs in der Messzelle (12) erhöht und abgesenkt wird und die Absorption in Abhängigkeit vom Druck gemessen wird, wobei zur Druckänderung ein Abfluss des Gasgemischs unterbrochen oder ein Zufluss oder ein Abfluss des Gasgemischs durch eine Pumpe (318) unterstützt und erhöht wird.

15. Verfahren zum Messen einer Gaskonzentration mittels Absorptionsspektroskopie, insbesondere zur kapnometrischen Messung des Anteils von $CO_2$ in Atemluft, nach Anspruch 14, **dadurch gekennzeichnet, dass** eine druckabhängige Messreihe in Bezug auf linear und nichtlinear vom Druck abhängige Anteile des Logarithmus des Messsignals analysiert wird und der nichtlinear vom Druck abhängige Anteil zur Messung der Gaskonzentration des zu messenden Gases oder zur Korrektur oder Kalibration einer Messung der Gaskonzentration des zu messenden Gases verwendet wird.

**Claims**

1. An arrangement (10, 110, 210, 310) for measuring a gas concentration by means of absorption spectroscopy, in particular for the capnometric measurement of the proportion of $CO_2$ in breathing air, which is designed in such a way that, in operation, IR light from a thermal light source is guided through a measuring cell (12) with a gas mixture to be analysed, and is further designed to determine the gas concentrations of a gas to be measured contained in the gas mixture via a measurement of an attenuation of the light introduced into the measuring cell (12) due to absorption in the gas to be measured, wherein the arrangement (10, 110, 210, 310) comprises an optical beam path with an IR light-generating thermal light source and an associated spherical or parabolic reflector (22), a measuring cell (12) which can be filled or is filled with the gas mixture, and a measuring path for the light generated by the thermal light source, at least one sensor (25, 27, 28, 28', 28", 30) and at least one band pass filter (24, 26, 212) which is connected upstream of the at least one sensor (25, 27, 28, 28', 28", 30), wherein the arrangement (10, 110, 210, 310) further comprises an evaluation device (18) designed to determine the gas concentration to be measured from the attenuation of the IR light in the measuring cell (12), wherein

   - at least two sensors are comprised and the at least one band-pass filter (24, 212) is designed for transmission in a measuring wavelength range in which the gas to be measured absorbs IR light, and the at least one band-pass filter (212) or at least one further band-pass filter (26) is designed for transmission in a reference wavelength range in which the gas to be measured does not absorb IR light or absorbs it only slightly in comparison with the measuring wavelength range, or
   - only a single sensor (30) is included and the band-pass filter connected upstream of the single sensor (30) is designed as a double band-pass filter (212) that transmits IR light both in the measurement wavelength range and in the reference wavelength range,

   wherein the thermal light source is designed as a micro filament lamp (20) with a light-generating filament (81) arranged in an evacuated capsule (84) or a capsule filled with an inert gas and is arranged at the focal point of the spherical or parabolic reflector (22), wherein the at least one sensor (25, 27, 28, 28', 28", 30) is designed as an infrared-sensitive photodiode whose sensitive area is less than 1 mm$^2$.

2. The arrangement (10, 110, 210, 310) according to claim 1, **characterized in that** the encapsulation (84) of the micro filament lamp (20) has a diameter (90) of less than 2 mm, in particular less than 1.5 mm, in particular less than 1 mm.

3. The arrangement (10, 110, 210, 310) according to claim 1 or 2, **characterized in that** a greatest linear distance between two points of the coil (81) is less than 1 mm, in particular less than 0.5 mm.

4. The arrangement (10, 110, 210, 310) according to one of claims 1 to 3, **characterized in that**, in a direction of projection in which the envelope assumes a maximum envelope projection surface, an envelope of the coil (81) has a maximum envelope projection surface of less than 0.1 mm$^2$, in particular less than 0.02 mm$^2$.

5. The arrangement (10, 110, 210, 310) according to one of claims 1 to 4, **characterized in that** the sensitive area of the sensor (25, 27, 28, 28', 28", 30) or sensors (25, 27, 28, 28', 28", 30) is less than 0.15 mm$^2$.

6. The arrangement (10, 110, 210, 310) according to one of claims 1 to 5, **characterized in that** a control device (60) is included that is designed for power-controlled actuation of the micro filament lamp (20), the control device (60), in order to determine an actual value of the power emitted, is designed to form a product of the current measured at the micro filament lamp (20) and the voltage measured, or is signal-connected to a photodiode (64) that is positioned at the micro filament lamp (20) and receives a portion of the light generated by the micro filament lamp (20).

7. The arrangement (10, 110, 210, 310) according to one of claims 1 to 6, **characterized in that** the IR light is passed through the measuring cell (12) in a bundled manner and, after passing through the measuring cell, is distributed to two or more sensors (25, 27, 28, 28', 28", 30) by means of a spectrally neutral optical transmission or reflection grating (40, 41, 42, 50-54), 28, 28', 28", 30), wherein the transmission grating (40, 41, 42) or reflection grating (50 - 54) has in particular a grating constant which is smaller by a factor of 30 or more, in particular by a factor of 50 and more, than a diameter of a light spot on the transmission or reflection grating (40, 41, 42, 50 - 54).

8. The arrangement (10, 110, 210, 310) according to one of claims 1 to 7, **characterized in that** the measuring cell (12) is designed as a tube that is diffuse or highly specular on the inside and at one end of which the micro filament lamp (20) is arranged and at the other end of which the sensor (25, 27, 28, 28', 28", 30) or the sensors (25, 27, 28, 28', 28", 30) with the preceding band pass filters (24, 26, 212) are arranged.

9. The arrangement (10, 110, 210, 310) according to any one of claims 1 to 8, **characterized in that**, if only a single sensor is comprised and the band pass filter preceding the single sensor is designed as a double band pass filter (212) that transmits IR light both in the measurement wavelength range and in the reference wavelength range, the control device (60) is designed and configured to modulate the micro filament lamp (20) between a low power operating point and a high power operating point at which the respective emission spectrum has different component ratios in the measurement wavelength range and in the reference wavelength range.

10. The arrangement (10, 110, 210, 310) according to one of claims 1 to 9, **characterized in that** means are included that are configured for temporarily increasing and reducing the pressure of the gas mixture in the measuring cell (12), in particular a pump (318) and/or one or more, in particular switchable, valves.

11. A method for measuring a gas concentration by means of absorption spectroscopy, in particular for the capnometric measurement of the proportion of $CO_2$ in breathing air, with an arrangement (10, 110, 210, 310) according to one of claims 1 to 10, in which IR light from the micro filament lamp (20) as thermal light source is passed through the measuring cell (12) with a gas mixture to be analysed, at least one measurement of an attenuation of the light introduced into the measuring cell (12) due to absorption in a gas to be measured contained in the gas mixture and at least one measurement of of a spectral reference by the at least one sensor (25, 27, 28, 28', 28", 30) is carried out, and from this the gas concentration of the gas to be measured is determined with the evaluation device (18) is determined.

12. The method according to claim 1, **characterized in that** the micro filament lamp (20) is modulated with a measurement repetition frequency $f_{Mess}$ that is greater than 10 Hz, in particular greater than 25 Hz, wherein a temperature of the coil (81) is greater than 400°C during measurement, and has a temperature modulation rise of at least 300°C, in particular at least 500°C, in particular exceeds 1000°C at a maximum.

13. The method according to claim 11 or 12, **characterized in that** the micro filament lamp (20) is operated with power control.

14. The method according to one of claims 11 to 13, **characterized in that**, over the course of measuring, the gas mixture pressure in the measuring cell (12) is increased and decreased successively in time intervals and the absorption is measured as a function of the pressure, wherein to change the pressure, in particular, an outflow of the gas mixture is interrupted, or an inflow or an outflow of the gas mixture is supported and increased by a pump (318).

15. A method for measuring a gas concentration by means of absorption spectroscopy, in particular for the capnometric measurement of the proportion of $CO_2$ in breathing air, according to claim 14, **characterised in that** a pressure-dependent series of measurements is analysed with respect to linear and non-linear pressure-dependent components of the logarithm of the measurement signal and the non-linear pressure-dependent component is used to measure the gas concentration of the gas to be measured or to correct or calibrate a measurement of the gas concentration of the gas to be measured.

**Revendications**

1. Agencement (10, 110, 210, 310) pour mesurer une concentration de gaz par spectroscopie d'absorption, en particulier pour la mesure capnométrique de la proportion de $CO_2$ dans l'air respiré, qui est conçu de telle sorte qu'en fonctionnement, de la lumière IR provenant d'une source de lumière thermique est guidée à travers une cellule de mesure (12) contenant un mélange gazeux à analyser, et est en outre conçu pour déterminer les concentrations de gaz d'un gaz à mesurer contenu dans le mélange gazeux par une mesure d'une atténuation de la lumière introduite dans la cellule de mesure (12) en raison de l'absorption dans le gaz à mesurer, l'agencement (10, 110, 210, 310) comprenant un trajet de faisceau optique avec une source de lumière thermique générant de la lumière IR et un réflecteur (22) sphérique ou parabolique associé, une cellule de mesure (12), apte à être remplie ou est remplie avec le mélange gazeux et un trajet de mesure pour la lumière générée par la source de lumière thermique, au moins un capteur (25, 27, 28, 28', 28", 30) ainsi qu'au moins un filtre passe-bande (24, 26, 212) qui est monté en amont dudit au moins un capteur (25, 27, 28, 28', 28", 30), l'agencement (10, 110, 210, 310) comprenant en outre un dispositif d'évaluation (18) qui est conçu pour déterminer la concentration de gaz à mesurer à partir de l'atténuation de la lumière IR dans la cellule de mesure (12),

   - au moins deux capteurs étant prévus et ledit au moins un filtre passe-bande (24, 212) étant conçu pour la transmission dans une plage de longueurs d'onde de mesure dans laquelle le gaz à mesurer absorbe la lumière IR, et ledit au moins un filtre passe-bande (212) ou au moins un autre filtre passe-bande (26) étant conçu pour la transmission dans une plage de longueurs d'onde de référence dans laquelle le gaz à mesurer n'absorbe pas la lumière IR ou l'absorbe seulement un peu par rapport à la plage de longueurs d'onde de mesure, ou
   - un capteur unique (30) étant seulement prévu et le filtre passe-bande placé en amont du capteur unique (30) étant conçu sous la forme d'un filtre passe-bande double (212) qui laisse passer la lumière IR aussi bien dans la plage de longueurs d'onde de mesure que dans la plage de longueurs d'onde de référence,

   la source de lumière thermique étant conçue sous la forme d'une micro-lampe à filament (20) avec un filament (81) générateur de lumière agencé dans une capsule (84) sous vide ou remplie d'un gaz protecteur et étant agencée au point focal du réflecteur (22) sphérique ou parabolique, ledit au moins un capteur (25, 27, 28, 28', 28", 30) étant conçu sous la forme d'une photodiode sensible à l'infrarouge, dont la surface sensible est inférieure à 1 mm$^2$.

2. Agencement (10, 110, 210, 310) selon la revendication 1, **caractérisé en ce que** la capsule (84) de la micro-lampe à filament (20) présente un diamètre (90) inférieur à 2 mm, notamment inférieur à 1,5 mm, en particulier inférieur à 1 mm.

3. Agencement (10, 110, 210, 310) selon la revendication 1 ou la revendication 2, **caractérisé en ce qu'**une distance linéaire maximale entre deux points du filament (81) est inférieure à 1 mm, notamment inférieure à 0,5 mm.

4. Agencement (10, 110, 210, 310) selon l'une des revendications 1 à 3, **caractérisé en ce qu'**une enveloppe du filament (81) présente, dans une direction de saillie dans laquelle l'enveloppe adopte une surface de saillie d'enveloppe maximale, une surface de saillie d'enveloppe maximale inférieure à 0,1 mm$^2$, notamment inférieure à 0,02 mm$^2$.

5. Agencement (10, 110, 210, 310) selon l'une des revendications 1 à 4, **caractérisé en ce que** la surface sensible du capteur (25, 27, 28, 28', 28", 30) ou des capteurs (25, 27, 28, 28', 28°, 30) est inférieure à 0,15 mm$^2$.

6. Agencement (10, 110, 210, 310) selon l'une quelconque des revendications 1 à 5, **caractérisé en ce qu'**il comprend un dispositif de commande (60) configuré pour commander la micro-lampe à filament (20) en fonction de la puissance, le dispositif de commande (60) étant conçu de façon à déterminer une valeur réelle de la puissance délivrée pour former un produit du courant mesuré et de la tension mesurée au niveau de la micro-lampe à filament (20), ou est relié par signal à une photodiode (64) disposée près de la micro-lampe à filament (20), qui reçoit une partie de la lumière produite par la micro-lampe à filament (20).

7. Agencement (10, 110, 210, 310) selon l'une des revendications 1 à 6, **caractérisé en ce que** la lumière IR est dirigée en faisceau à travers la cellule de mesure (12) et, après avoir traversé la cellule de mesure, est répartie sur deux ou plusieurs capteurs (25, 27, 28, 28', 28°, 30) au moyen d'un réseau de transmission ou de réflexion optique (40, 41, 42, 50 - 54) plan-parallèle ou courbe, neutre du point de vue spectral, le réseau de transmission (40, 41, 42) ou de réflexion (50-54) présentant une constante de réseau qui est inférieure d'un facteur de 30 ou plus, en particulier d'un facteur de 50 et plus, à un diamètre d'un point lumineux sur le réseau de transmission ou de réflexion (40, 41, 42, 50-54).

8. Agencement (10, 110, 210, 310) selon l'une des revendications 1 à 7, **caractérisé en ce que** la cellule de mesure (12) est conçue sous la forme d'un tube à effet miroir diffus ou brillant à l'intérieur, à une extrémité duquel est agencée la micro-lampe à filament (20) et à l'autre extrémité duquel est agencé le capteur (25, 27, 28, 28', 28°, 30) ou les capteurs (25, 27, 28, 28', 28", 30) avec les filtres passe-bande (24, 26, 212) en amont.

9. Agencement (10, 110, 210, 310) selon l'une des revendications 1 à 8, **caractérisé en ce que**, lorsqu'il ne comprend qu'un seul capteur et que le filtre passe-bande placé en amont du capteur unique est conçu sous la forme d'un filtre passe-bande double (212) qui laisse passer la lumière IR à la fois dans la plage de longueurs d'onde de mesure et dans la plage de longueurs d'onde de référence, le dispositif de commande (60) est conçu et agencé pour moduler la micro-lampe à filament (20) entre un point de fonctionnement à faible puissance et un point de fonctionnement à puissance élevée, dans lesquels le spectre d'émission respectif présente des proportions différentes dans la plage de longueurs d'onde de mesure et dans la plage de longueurs d'onde de référence.

10. Agencement (10, 110, 210, 310) selon l'une des revendications 1 à 9, **caractérisé en ce qu'**il comprend des moyens conçus pour augmenter et diminuer temporairement la pression du mélange gazeux dans la cellule de mesure (12), en particulier une pompe (318) et/ou une ou plusieurs soupapes, en particulier commutables.

11. Procédé de mesure d'une concentration de gaz par spectroscopie d'absorption, en particulier pour la mesure capnométrique de la proportion de $CO_2$ dans l'air respiré, avec un agencement (10, 110, 210, 310) selon l'une des revendications 1 à 10, dans lequel de la lumière IR provenant de la lampe à filament (20) micro-filamentaire est guidée en tant que source de lumière thermique à travers la cellule de mesure (12) avec un mélange gazeux à analyser, au moins une mesure d'une atténuation de la lumière introduite dans la cellule de mesure (12) en raison de l'absorption dans un gaz à mesurer contenu dans le mélange gazeux et au moins une mesure d'une référence spectrale est effectuée par ledit au moins un capteur (25, 27, 28, 28', 28", 30) et la concentration en gaz du gaz à mesurer est déterminée à partir du dispositif d'évaluation (18).

12. Procédé selon la revendication 11, **caractérisé en ce que** la micro-lampe à filament (20) est modulée à une fréquence de répétition de mesure $f_{Mess}$ supérieure à 10 Hz, notamment supérieure à 25 Hz, une température du filament (81) étant supérieure à 400°C pendant la mesure et présentant une amplitude de modulation de température d'au moins 300°C, notamment d'au moins 500°C, notamment supérieure à 1000°C au maximum.

13. Procédé selon la revendication 11 ou la revendication 12, **caractérisé en ce que** la micro-lampe à filament (20) est exploitée avec régulation de puissance.

14. Procédé selon l'une des revendications 11 à 13, **caractérisé en ce que**, au cours d'une mesure, on augmente et on diminue à intervalles de temps successifs la pression du mélange gazeux dans la cellule de mesure (12) et l'absorption est mesurée en fonction de la pression, un écoulement du mélange gazeux étant interrompu pour modifier la pression ou un afflux ou un écoulement du mélange gazeux étant soutenu et augmenté par une pompe (318).

15. Procédé de mesure d'une concentration de gaz par spectroscopie d'absorption, en particulier pour la mesure capnométrique de la proportion de $CO_2$ dans l'air respirable, selon la revendication 14, **caractérisé en ce qu'**une série de mesures dépendant de la pression est analysée en ce qui concerne les parties du logarithme du signal de mesure qui dépendent linéairement et non linéairement de la pression, et la partie qui dépend non linéairement de la pression est utilisée pour mesurer la concentration de gaz du gaz à mesurer ou pour corriger ou calibrer une mesure de la concentration de gaz du gaz à mesurer.

Fig. 1

Fig. 2

Fig. 3

Fig. 4

a) ~50

b) ~51

c) ~52

53
d)

54
e)

54
f)

Fig. 5

110

20
22
12
112

A:A

53    24   32
25

114

A:A

25
32
24
26
27

112

53

114'

Fig. 6

Fig. 7

Fig. 8

Fig. 9

Fig. 10

Fig. 11

a)

b)

c)

d)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 03060490 A
- US 5464982 A
- US 5092342 A
- US 4618771 A
- US 6277081 B1
- WO 2007091043 A1
- KR 20160063704 A
- US 5445160 A
- US 20110147592 A1

**In der Beschreibung aufgeführte Nicht-Patentliteratur**

- **AUS WANG, X. et al.** High Performance CO2 Measurement Based on Pressure Modulation. *Procedia Engineering,* 2010, vol. 5, 1208-1211